# EUROPÄISCHE PATENTSCHRIFT

(11) **EP 1 406 610 B1**
(45) Veröffentlichungstag und Bekanntmachung des Hinweises auf die Patenterteilung: **26.01.2005**
(21) Anmeldenummer: 02754819.7
(22) Anmeldetag: 03.07.2002
(51) Int. Cl.: A61K 31/365, A61K 31/416, A61K 31/4155, A61K 31/443, A61K 31/4709, A61K 31/473, A61K 31/497, A61K 31/498, A61K 31/506, A61K 31/517, A61P 3/10, A61P 25/16, A61P 25/28, A61P 31/10, A61P 35/00, A61P 29/00

(54) **VERWENDUNG VON SUBSTITUIERTEN GAMMA-LACTONVERBINDUNGEN ALS ARZNEIMITTEL**
USE OF SUBSTITUTED GAMMA-LACTONE COMPOUNDS AS MEDICAMENTS
UTILISATION DE COMPOSES DE GAMMA-LACTONE SUBSTITUES EN TANT QUE MEDICAMENTS

(30) Priorität: 05.07.2001 DE 10132726
(43) Veröffentlichungstag der Anmeldung: 14.04.2004
(73) Patentinhaber: Grünenthal GmbH, 52078 Aachen (DE)
(72) Erfinder: Sundermann, Corinna, 52066 Aachen (DE); SUNDERMANN, Bernd, 52066 Aachen (DE); PRZEWOSNY, Michael, 52062 Aachen (DE); HENNIES, Hagen-Heinrich, 52152 Simmerath (DE)
(74) Vertreter: Kutzenberger, Helga, Dr.
(86) Internationale Anmeldenummer: PCT/EP2002/007382
(87) Internationale Veröffentlichungsnummer: WO 2003/004016

(56) Entgegenhaltungen:
- WO-A-91/02725
- MURAD F: "Discovery of Some of the Biological Effetcs of Nitric Oxide and Its Role in Cell Signalling (Nobel Lecture)" ANGEWANDTE CHEMIE. INTERNATIONAL EDITION, VERLAG CHEMIE. WEINHEIM, DE, Bd. 38, 1999, Seiten 1857-1868, XP002159759 ISSN: 0570-0833

## Beschreibung

Die vorliegende Erfindung betrifft die Verwendung von substituierten γ-Lactonverbindungen zur Herstellung von Arzneimitteln zur Behandlung von Migräne, septischem Schock, neurodegenerativen Krankheiten, wie multipler Sklerose, Morbus Parkinson, Morbus Alzheimer oder Morbus Huntington, Entzündungen, Entzündungsschmerz, cerebraler Ischämie, Diabetes, Meningitis, Arteriosklerose, Krebserkrankungen, Pilzerkrankungen oder zur Wundheilung.

Stickstoffmonoxid (NO) reguliert zahlreiche physiologische Prozesse, unter anderem die Neurotransmission, die Relaxation und Proliferation von glatter Muskulatur, die Adhäsion und Aggregation von Thrombozyten sowie die Gewebeverletzung und Entzündung. Aufgrund der Vielzahl von Signalfunktionen wird Stickstoffmonoxid mit einer Reihe von Krankheiten in Verbindung gebracht, beispielsweise in L. J. Ignarro, Angew. Chem. (1999), 111, Seiten 2002-2013 und in F. Murad, Angew. Chem. Int. Ed. (1999), 111, Seiten 1976-1989. Eine wichtige Rolle bei der therapeutischen Beeinflussung dieser Krankheiten spielt dabei das für die physiologische Bildung von Stickstoffmonoxid verantwortliche Enzym, die Stickstoffmonoxid-Synthase (NO-Synthase). Bislang wurden drei verschiedene Isoformen der NO-Synthase identifiziert, nämlich die beiden konstitutiven Formen nNO-Synthase und eNO-Synthase sowie die induzierbare Form iNO-Synthase (A. J. Hobbs, A. Higgs, S. Moncada, Annu. Rev. Pharmacol. Toxicol. (1999), 39, Seiten 191-220; I. C. Green, P.-E. Chabrier, DDT (1999), 4, Seiten 47-49; P.-E. Chabrier et al., Cell. Mol. Life Sci. (1999), 55, Seiten 1029-1035).

Die Hemmung der NO-Synthase eröffnet neue Therapieansätze für verschiedene Krankheiten, die mit Stickstoffmonoxid in Zusammenhang stehen (A. J. Hobbs et al., Annu. Rev. Pharmacol. Toxicol. (1999), 39, Seiten 191-220; I. C. Green, P.-E. Chabrier, DDT (1999), 4, Seiten 47-49; P.-E. Chabrier et al., Cell. Mol. Life Sci. (1999), 55, Seiten 1029-1035), wie beispielsweise Migräne (L. L. Thomsen, J. Olesen, Clinical Neuroscience (1998), 5, Seiten 28-33; L. H. Lassen et al., The Lancet (1997), 349, 401-402), septischer Schock, neurodegenerative Erkrankungen wie Multiple Sklerose; Morbus Parkinson, Morbus Alzheimer oder Morbus Huntington, Entzündungen, Entzündungsschmerz, cerebrale Ischämie, Diabetes, Meningitis und Arteriosklerose.

Darüber hinaus kann die Inhibierung der NO-Synthase einen Effekt auf die Wundheilung, auf Tumore und auf die Angiogenese haben sowie eine unspezifische Immunität gegen Mikroorganismen bewirken (A. J. Hobbs et al., Annu. Rev. Pharmacol. Toxicol. (1999), 39, Seiten 191-220).

Bislang bekannte Wirkstoffe, die die NO-Synthase hemmen, sind neben L-NMMA und L-NAME - d.h. Analoga des L-Arginins, aus dem in-vivo unter Beteiligung von NO-Synthase Stickstoffmonoxid und Citrullin gebildet werden - u.a. S-Methyl-L-citrullin, Aminoguanidin, S-Methylisoharnstoff, 7-Nitroindazol und 2-Mercaptoethylguanidin (A. J. Hobbs et al., Annu. Rev. Pharmacol. Toxicol. (1999), 39, Seiten 191-220).

Darüber hinaus besteht ein Bedarf nach weiteren entsprechenden Wirkstoffen.

Eine Aufgabe der vorliegenden Erfindung bestand daher darin, Wirkstoffe zur Verfügung zu stellen, die als Inhibitor auf die Stickstoffmonoxid-Synthase wirken. Insbesondere sollen sich die Arzneimittel, die solche Wirkstoffe enthalten, zur Behandlung von Migräne, septischem Schock, neurodegenerativer Krankheiten, wie Multipler Sklerose, Morbus Parkinson, Morbus Alzheimer oder Morbus Huntington, Entzündungen, Entzündungsschmerz, cerebraler Ischämie, Diabetes, Meningitis, Arteriosklerose, Krebserkrankungen, Pilzerkrankungen oder zur Wundheilung eignen.

Überraschenderweise wurde nun gefunden, daß substituierte γ-Lactonverbindungen der nachstehenden allgemeinen Formel I als Inhibitoren auf die Stickstoffmonoxid-Synthase wirken und sich insbesondere zur Herstellung eines Arzneimittels zur Behandlung von Migräne, septischem Schock, neurodegenerativen Erkrankungen, wie Multipler Sklerose, Morbus Parkinson, Morbus Alzheimer oder Morbus Huntington, Entzündungen, Entzündungsschmerz, cerebraler Ischämie, Diabetes, Meningitis, Arteriosklerose, Krebserkrankungen, Pilzerkrankungen oder zur Wundheilung eignen.

Ein Gegenstand der vorliegenden Erfindung ist daher die Verwendung wenigstens einer substituierten γ-Lactonverbindung der allgemeinen Formel I 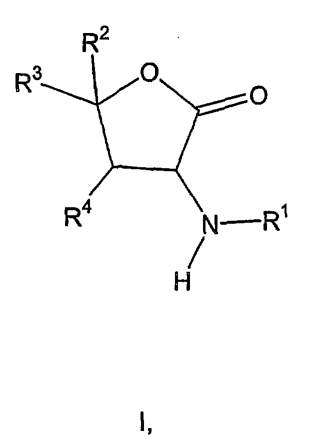 worin
R¹ für einen gegebenenfalls wenigstens einfach substituierten 2-Pyridyl-, 2-Pyrimidyl-, 2-Pyrazolyl-, 2-Chinolinyl- oder 2-Pyrazinyl-Rest steht, der auch mit einem gesättigten oder zumindest teilweise ungesättigten Kohlenwasserstoff-Ringsystem kondensiert sein kann, vorzugsweise für einen gegebenenfalls wenigstens einfach substituierten 2-Pyridyl-Rest steht, der auch mit einem gesättigten oder zumindest teilweise ungesättigten Kohlenwasserstoff-Ringsystem kondensiert sein kann, besonders bevorzugt für einen wenigstens in 4-Stellung substituierten 2-Pyridyl-Rest steht,
R² für einen gegebenenfalls wenigstens einfach substituierten, gesättigten, verzweigten oder unverzweigten aliphatischen C₁₋₁₀-Rest oder für einen gegebenenfalls wenigstens einfach substituierten, zumindest teilweise ungesättigten, verzweigten oder unverzweigten aliphatischen C₂₋₁₀-Rest, vorzugsweise für einen gegebenenfalls wenigstens einfach substituierten, verzweigten oder unverzweigten C₁₋₆-Alkyl-Rest, steht,
R³ für einen gegebenenfalls wenigstens einfach substituierten Aryl-Rest steht,
R⁴ für H steht,
oder
R³ und R⁴ zusammen für einen gegebenenfalls wenigstens einfach substituierten, gesättigten oder wenigstens einfach ungesättigten aliphatischen C₃₋₇-Rest stehen, mit der Maßgabe, daß der Rest R² in diesem Fall für einen gegebenenfalls wenigstens einfach substituierten Aryl-Rest, für einen einen gegebenenfalls wenigstens einfach substituierten, gesättigten, verzweigten oder unverzweigten aliphatischen C₁₋₁₀-Rest oder für einen gegebenenfalls wenigstens einfach substituierten, zumindest teilweise ungesättigten, verzweigten oder unverzweigten aliphatischen C₂₋₁₀-Rest steht,
in Form ihrer Racemate, Diastereomeren oder Enantiomeren als freie Base oder eines entsprechenden physiologisch verträglichen Salzes zur Herstellung eines Arzneimittels zur Behandlung von Migräne, septischem Schock, neurodegenerativen Erkrankungen, vorzugsweise Multipler Sklerose, Morbus Parkinson, Morbus Alzheimer oder Morbus Huntington, Entzündungen, Entzündungsschmerz, cerebraler Ischämie, Diabetes, Meningitis, Arteriosklerose, Krebserkrankungen, Pilzerkrankungen oder zur Wundheilung.

Bevorzugt ist die Verwendung wenigstens einer substituierten γ-Lactonverbindung der oben angegebenen allgemeinen Formel I, worin R³ für einen gegebenenfalls wenigstens einfach substituierten Aryl-Rest und R⁴ für H steht und die übrigen Reste R¹ und R² die Bedeutung gemäß der oben angegebenen allgemeinen Formel I haben.

Die aliphatischen Reste können einfach oder mehrfach substituiert sein. Sofern die aliphatischen Reste mehr als einen Substituenten aufweisen, können diese gleich oder verschieden sein und sowohl an dem selben wie auch an verschiedenen Atomen des aliphatischen Restes gebunden sein.
Bevorzugt ist der aliphatische Rest ausgewählt aus der Gruppe bestehend aus gegebenenfalls wenigstens einfach substituiertem Methyl, Ethyl, Propyl, Isopropyl, n-Butyl, sek-Butyl, tert-Butyl, n-Pentyl, Neopentyl, n-Hexyl, n-Heptyl, n-Octyl, n-Nonyl, n-Decyl, Propenyl, Butenyl, Pentenyl, Hexenyl, Heptenyl, Octenyl, Propinyl, Butinyl, Pentinyl, Hexinyl, Heptinyl und Octinyl. Die Substituenten sind vorzugsweise ausgewählt aus der Gruppe bestehend aus F, Cl, Br, I, NH₂, SH und OH.

Unter einem Aryl-Rest werden im Sinne der vorliegenden Erfindung auch solche aromatischen Kohlenwasserstoffreste verstanden, die mit einem gesättigten oder zumindest teilweise ungesättigten Kohlenwasserstoff-Ringsystem kondensiert sind.
Bevorzugt ist als Aryl-Rest ein gegebenenfalls wenigstens einfach substituierter Phenyl-, Naphthyl- oder Anthracenyl-Rest, besonders bevorzugt ein gegebenenfalls einfach substituierter Phenyl-Rest.
Sofern der Aryl-Rest mehr als einen Substituenten aufweist, können diese gleich oder verschieden sein. Vorzugsweise sind die Substituenten ausgewählt aus der Gruppe bestehend aus F, Cl, Br, I, NH₂, SH, OH, CF₃, CN, NO₂, OR⁵, SR⁵, NR⁶R⁷ und unsubstituiertem oder wenigstens einfach mit F, Cl, Br, I, NH₂, SH, OH, CF₃, CN oder NO₂ substituiertem C₁₋₆-Alkyl, C₁₋₆-Alkoxy, C₂₋₈-Alkenyl, C₂₋₈-Alkinyl, Phenyl, Phenoxy und Benzyloxy, wobei
R⁵ für H, einen C₁₋₁₀-Alkyl-Rest, einen unsubstituierten Aryl- oder Heteroaryl-Rest oder für einen unsubstituierten, über eine C₁₋₃-Alkylen-Gruppe gebundenen Aryl- oder Heteroaryl-Rest steht und
R⁶ und R⁷, gleich oder verschieden, für H, einen C₁₋₁₀-Alkyl-Rest, einen unsubstituierten Aryl- oder Heteroaryl-Rest oder für einen unsubstituierten, über eine C₁₋₃-Alkylen-Gruppe gebundenen Aryl- oder Heteroaryl-Rest stehen.

Sofern der Rest R¹ für einen einfach oder mehrfach substituierten, ggf. mit einem gesättigten oder zumindest teilweise ungesättigten Kohlenwasserstoff-Ringsystem kondensierten 2-Pyridyl-, 2-Pyrimidyl-, 2-Pyrazolyl-, 2-Chinolinyloder 2-Pyrazinyl-Rest steht, können die Substituenten gleich oder verschieden sein und bevorzugt ausgewählt werden aus der Gruppe bestehend aus F, Cl, Br, I, NH₂, SH, OH, CF₃, CN, NO₂, OR⁵, SR⁵, NR⁶R⁷ und unsubstituiertem oder wenigstens einfach mit F, Cl, Br, I, NH₂, SH, OH, CF₃, CN oder NO₂ substituiertern C₁₋₆-Alkyl, C₁₋₆-Alkoxy, C₂₋₈-Alkenyl, C₂₋₈-Alkinyl, Phenyl, Phenoxy und Benzyloxy, wobei die Reste R⁵ R⁶ und R⁷ die oben angegebene Bedeutung haben.

Unter einem Heteroaryl-Rest werden im Sinne der vorliegenden Erfindung auch solche heteroaromatischen Kohlenwasserstoffreste verstanden, die mit einem gesättigten oder zumindest teilweise ungesättigten Kohlenwasserstoff-Ringsystem kondensiert sind. Vorzugsweise enthält der Heteroaryl-Rest ein Heteroatom ausgewählt aus der Gruppe bestehend aus Schwefel, Stickstoff und Sauerstoff. Bevorzugt ist der Heteroaryl-Rest ein unsubstituierter Thiophenyl-, Furanyl-, Pyrrolyl-, Pyridinyl-, Pyrimidinyl-, Chinolinyl-, Isochinolinyl-, Phthalazinyl- oder Chinazolinyl-Rest.

Besonders bevorzugt ist die Verwendung wenigstens einer der nachfolgenden Verbindungen der allgemeinen Formel I:
5-(2,4-Dimethyl-phenyl)-3-(8-hydroxy-chinolin-2-ylamino)-5-methyl-dihydrofuran-2-on,
5-(3,4-Dimethyl-phenyl)-3-(4,6-dimethyl-pyridin-2-ylamino)-5-methyl-dihydrofuran-2-on,
5-(2,4-Dimethyl-phenyl)-3-(4,6-dimethyl-pyridin-2-ylamino)-5-methyl-dihydrofuran-2-on,
5-(4-Cyclohexyl-phenyl)-3-(4,6-dimethyl-pyridin-2-ylamino)-5-methyl-dihydrofuran-2-on,
5-(3,5-Dimethyl-phenyl)-3-(4,6-dimethyl-pyridin-2-ylamino)-5-methyl-dihydrofuran-2-on,
5-(3,4-Dimethyl-phenyl)-5-methyl-3-(4-methyl-pyridin-2-ylamino)-dihydro-furan-2-on,
5-(2,4-Dimethyl-phenyl)-5-methyl-3-(4-methyl-pyridin-2-ylamino)-dihydro-furan-2-on,
5-(4-Cyclohexyl-phenyl)-5-methyl-3-(4-methyl-pyridin-2-ylamino)-dihydro-furan-2-on,
5-Methyl-3-(chinolin-2-ylamino)-5-m-tolyl-dihydro-furan-2-on,
3-(4,6-Dimethyl-pyridin-2-ylamino)-5-methyl-5-p-tolyl-dihydro-furan-2-on,
3-(4,6-Dimethyl-pyridin-2-ylamino)-5-methyl-5-m-tolyl-dihydro-furan-2-on,
3-(4,6-Dimethyl-pyridin-2-ylamino)-5-(4-ethoxy-phenyl)-5-methyl-dihydro-furan-2-on,
4-[4-(3-Brom-5-methyl-pyridin-2-ylamino)-2-methyl-5-oxo-tetrahydro-furan-2-yl]-benzonitril,
3-(3-Brom-5-methyl-pyridin-2-ylamino)-5-(4-tert-butyl-phenyl)-5-methyl-dihydrofuran-2-on,
5-(4-tert-Butyl-phenyl)-5-methyl-3-(6-propyl-pyridin-2-ylamino)-dihydro-furan-2-on,
5-(4-tert-Butyl-phenyl)-5-methyl-3-(4-methyl-3-nitro-pyridin-2-ylamino)-dihydrofuran-2-on,
3-(5-Brom-6-methyl-pyridin-2-ylamino)-5-methyl-5-(4-phenoxy-phenyl)-dihydrofuran-2-on,
5-(4-tert-Butyl-phenyl)-5-methyl-3-(3-nitro-pyridin-2-ylamino)-dihydro-furan-2-on,
3-(3-Benzyloxy-pyridin-2-ylamino)-5-methyl-5-(4-phenoxy-phenyl)-dihydrofuran-2-on,
3-(3-Benzyloxy-pyridin-2-ylamino)-5-(4-tert-butyl-phenyl)-5-methyl-dihydrofuran-2-on,
3-(4,6-Dimethyl-pyridin-2-ylamino)-5-methyl-5-(4-phenoxy-phenyl)-dihydrofuran-2-on,
5-(4-tert-Butyl-phenyl)-3-(4,6-dimethyl-pyridin-2-yl-amino)-5-methyldihydrofuran-2-on
5-Methyl-3-(4-methyl-pyridin-2-ylamino)-5-(4-phenoxy-phenyl)-dihydro-furan-2-on,
5-(4-tert-Butyl-phenyl)-5-methyl-3-(4-methyl-pyridin-2-ylamino)-dihydro-furan-2-on,
4-[4-(5-Brom-3-nitro-pyridin-2-ylamino)-2-methyl-5-oxo-tetrahydro-furan-2-yl]-benzonitril,
4-[4-(5-Brom-pyrimidin-2-ylamino)-2-methyl-5-oxo-tetrahydro-furan-2-yl]-benzonitril,
5-Benzo[b]thiophen-2-yl-5-methyl-3-(6-propyl-pyridin-2-ylamino)-dihydro-furan-2-on,
3-(4,6-Dimethyl-pyridin-2-ylamino)-5-(4-isopropyl-phenyl)-5-methyl-dihydrofuran-2-on,
5-Benzofuran-2-yl-3-(4,6-dimethyl-pyridin-2-ylamino)-5-methyl-dihydro-furan-2-on,
5-Benzo[b]thiophen-2-yl-5-methyl-3-(4-methyl-pyridin-2-ylamino)-dihydro-furan-2-on,
5-Benzofuran-2-yl-5-methyl-3-(4-methyl-pyridin-2-ylamino)-dihydro-furan-2-on,
3-(5-Benzo[1,3]dioxol-5-yl-5-methyl-2-oxo-tetrahydro-furan-3-ylamino)-1Hpyrazol-4-carbonitril,
3-(5-Benzo[1,3]dioxol-5-yl-5-methyl-2-oxo-tetrahydro-furan-3-ylamino)-1Hpyrazol-4-carbonsäureethylester,
5-Benzo[1,3]dioxol-5-yl-5-methyl-3-(3-nitro-pyridin-2-ylamino)-dihydro-furan-2-on,
3-(4,6-Dimethyl-pyridin-2-ylamino)-5-methyl-5-(5,6,7,8-tetrahydro-naphthalin-2-yl)-dihydro-furan-2-on,
3-(4,6-Dimethyl-pyridin-2-ylamino)-5-methyl-5-naphthalin-2-yl-dihydro-furan-2-on,
5-Benzo[1,3]dioxol-5-yl-5-methyl-3-(4-methyl-pyridin-2-ylamino)-dihydro-furan-2-on,
5-Methyl-3-(4-methyl-pyridin-2-ylamino)-5-(5,6,7,8-tetrahydro-naphthalin-2-yl)-dihydro-furan-2-on,
5-Benzo[1,3]dioxol-5-yl-5-methyl-3-(5-methyl-pyridin-2-ylamino)-dihydro-furan-2-on,
5-Benzo[1,3]dioxol-5-yl-5-methyl-3-(6-methyl-pyridin-2-ylamino)-dihydro-furan-2-on,
3-(5-Brom-3-nitro-pyridin-2-ylamino)-5-methyl-5-(5,6,7,8-tetrahydro-naphthalin-2-yl)-dihydro-furan-2-on,
3-(5-Brom-3-nitro-pyridin-2-ylamino)-5-isopropyl-5-phenyl-dihydro-furan-2-on,
5-Isopropyl-3-(5-nitro-pyridin-2-ylamino)-5-phenyl-dihydro-furan-2-on,
5-Methyl-5-naphthalin-2-yl-3-(5-nitro-pyridin-2-ylamino)-dihydro-furan-2-on,
5-Isopropyl-5-phenyl-3-(pyrimidin-2-ylamino)-dihydro-furan-2-on,
3-[5-(4-lod-phenyl)-5-methyl-2-oxo-tetrahydro-furan-3-ylamino]-1H-pyrazol-4-carbonsäureethylester,
5-(4-Brom-phenyl)-3-(5-brom-pyridin-2-ylamino)-5-methyl-dihydro-furan-2-on,
3-(3-Brom-5-methyl-pyridin-2-ylamino)-5-methyl-5-naphthalin-1-yl-dihydrofuran-2-on,
5-Methyl-5-naphthalin-1-yl-3-(6-propyl-pyridin-2-ylamino)-dihydro-furan-2-on,
5-(3-Chlor-phenyl)-5-methyl-3-(6-propyl-pyridin-2-ylamino)-dihydro-furan-2-on,
5-(3-Chlor-phenyl)-5-methyl-3-(4-methyl-3-nitro-pyridin-2-ylamino)-dihydrofuran-2-on,
5-(4-Brom-phenyl)-5-methyl-3-(4-methyl-3-nitro-pyridin-2-ylamino)-dihydrofuran-2-on,
3-(5-Brom-6-methyl-pyridin-2-ylamino)-5-methyl-5-naphthalin-1 -yl-dihydrofuran-2-on,
3-(6-Brom-6-methyl-pyridin-2-ylamino)-5-(4-iodo-phenyl)-5-methyl-dihydrofuran-2-on,
3-(3-Benzyloxy-pyridin-2-ylamino)-5-(4-iodo-phenyl-5-methyl-dihydro-furan-2-on,
3-(3-Benzyloxy-pyridin-2-ylamino)-5-(4-brom-phenyl)-5-methyl-dihydro-furan-2-on,
3-(4,6-Dimethyl-pyridin-2-ylamino)-5-(4-iodo-phenyl)-5-methyl-dihydro-furan-2-on,
5-(3-Chlor-phenyl)-3-(4,6-dimethyl-pyridin-2-ylamino)-5-methyl-dihydro-furan-2-on,
5-(4-Brom-phenyl)-5-methyl-3-(3-methyl-pyridin-2-ylamino)-dihydro-furan-2-on,
5-(4-Brom-phenyl)-5-methyl-3-(4-methyl-pyridin-2-ylamino)-dihydro-furan-2-on,
2-[5-(3,5-Dimethoxy-phenyl)-5-methyl-2-oxo-tetrahydro-furan-3-ylamino]-4-propyl-pyrimidin-5-carbonsäureethylester,
3-(4-Brom-1H-pyrazol-3-ylamino)-5-(3,5-dimethoxy-phenyl)-5-methyl-dihydrofuran-2-on,
3-(4-Brom-1H-pyrazol-3-ylamino)-5-(2-methoxy-phenyl)-5-methyl-dihydro-furan-2-on,
3-[5-(2,5-Dimethoxy-phenyl)-5-methyl-2-oxo-tetrahydro-furan-3-ylamino]-1Hpyrazol-4-carbonitril,
3-[5-(2,5-Dimethoxy-phenyl)-5-methyl-2-oxo-tetrahydro-furan-3-ylamino]-5-methylsulfanyl-1H-pyrazol-4-carbonitril,
5-(2,5-Dimethoxy-phenyl)-5-methyl-3'-(pyridin-2-ylamino)-dihydro-furan-2-on,
5-(2-Methoxy-phenyl)-5-methyl-3-(pyridin-2-ylamino)-dihydro-furan-2-on,
3-(3-Chlor-5-trifluormethyl-pyridin-2-ylamino)-5-(3,5-dimethoxy-phenyl)-5-methyl-dihydro-furan-2-on,
3-(3-Chlor-5-trifluormethyl-pyridin-2-ylamino)-5-(2,5-dimethoxy-phenyl)-5-methyl-dihydro-furan-2-on,
3-(3,5-Dichlor-pyridin-2-ylamino)-5-(2-methoxy-phenyl)-5-methyl-dihydro-furan-2-on,
3-(3-Chlor-5-trifluormethyl-pyridin-2-ylamino-5-(2,4-dimethoxy-phenyl)-5-methyl-dihydro-furan-2-on,
3-(4,6-Dimethyl-pyridin-2-ylamino)-5-(3-methoxy-phenyl)-5-methyl-dihydrofuran-2-on,
3-(4,6-Dimethyl-pyridin-2-ylamino)-5-(4-methoxy-phenyl)-5-methyl-dihydrofuran-2-on,
5-(3,4-Dimethoxy-phenyl)-3-(4,6-dimethyl-pyridin-2-ylamino)-5-methyl-dihydrofuran-2-on,
5-(4-Methoxy-phenyl)-5-methyl-3-(4-methyl-pyridin-2-ylamino)-dihydro-furan-2-on,
5-(2,5-Dimethoxy-phenyl)-5-methyl-3-(pyrazin-2-ylamino)-dihydro-furan-2-on,
5-Methylsulfanyl-3-(2-oxo-5-phenyl-5-propyl-tetrahydro-furan-3-ylamino)-1Hpyrazol-4-carbonitril
oder eines entsprechenden physiologisch verträglichen Salzes, vorzugsweise des entsprechenden Hydrochlorids.

Vorzugsweise kann die Herstellung der erfindungsgemäß zum Einsatz kommenden substituierten γ-Lactonverbindungen der oben angegebenen allgemeinen Formel I wie nachstehend beschrieben erfolgen:

Bevorzugt wird zur Herstellung der erfindungsgemäß zum Einsatz kommenden γ-Lactonverbindungen der allgemeinen Formel I, worin die Reste R¹ bis R⁴ die oben angegebene Bedeutung haben, wenigstens eine Aminkomponente der allgemeinen Formel II 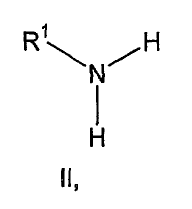 worin der Rest R¹ die oben angegebene Bedeutung hat, mit Glyoxalsäure, vorzugsweise in Form des Monohydrats oder einer wäßrigen Lösung,
und wenigstens einer Alkenkomponente der allgemeinen Formel III 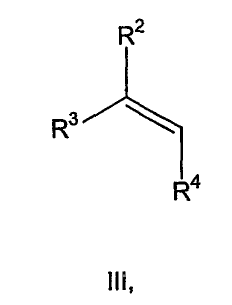 worin die Reste R² bis R⁴ die oben angegebene Bedeutung haben, in Gegenwart zumindest einer organischen und/oder anorganischen Säure, vorzugsweise Trifluoressigsäure in einem organischen Lösungsmittel zu wenigstens einer Verbindung der oben angegebenen allgemeinen Formel I umgesetzt und diese gegebenenfalls nach üblichen Methoden gereinigt und/oder gegebenenfalls nach üblichen Methoden isoliert.

Die Mengen der jeweils einzusetzenden Reaktionskomponenten sowie der anorganischen und/oder organischen Säure, die Temperatur während der Umsetzung und die Dauer der Umsetzung können variieren. Die für die jeweilige Umsetzung geeignete Menge der einzusetzenden Komponenten, die geeignete Temperatur sowie die geeignete Dauer der Umsetzung können vom Fachman durch einfach Vorversuche ermittelt werden. Vorzugsweise beträgt die Temperatur während der Umsetzung 0 bis 100 °C, besonders bevorzugt 15 bis 40 °C. Die Dauer der Umsetzung beträgt vorzugsweise 0,25 bis 12 Stunden.

Als geeignetes Lösungsmittel wird bevorzugt Acetonitril oder ein Gemisch enthaltend Acetonitril eingesetzt.

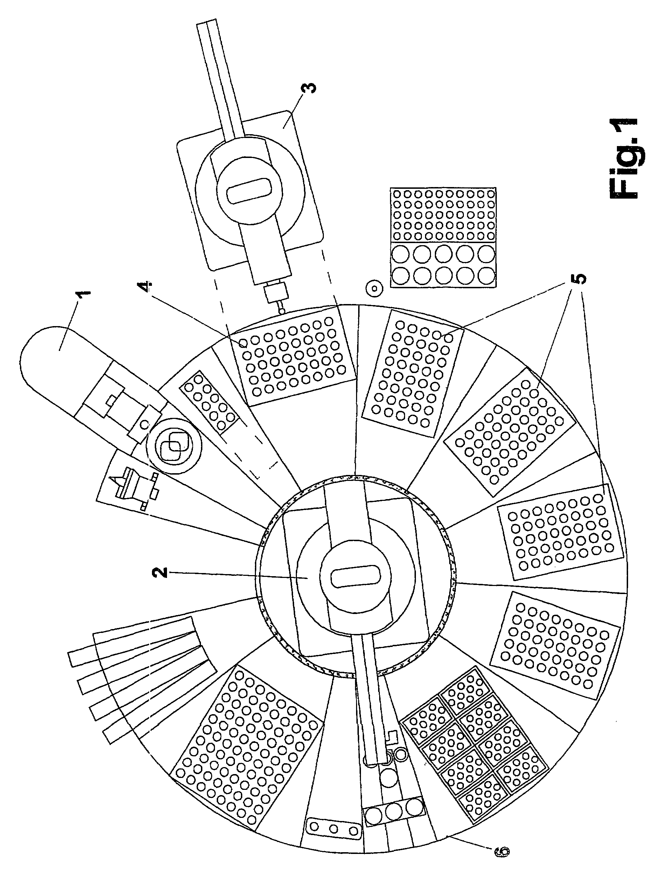

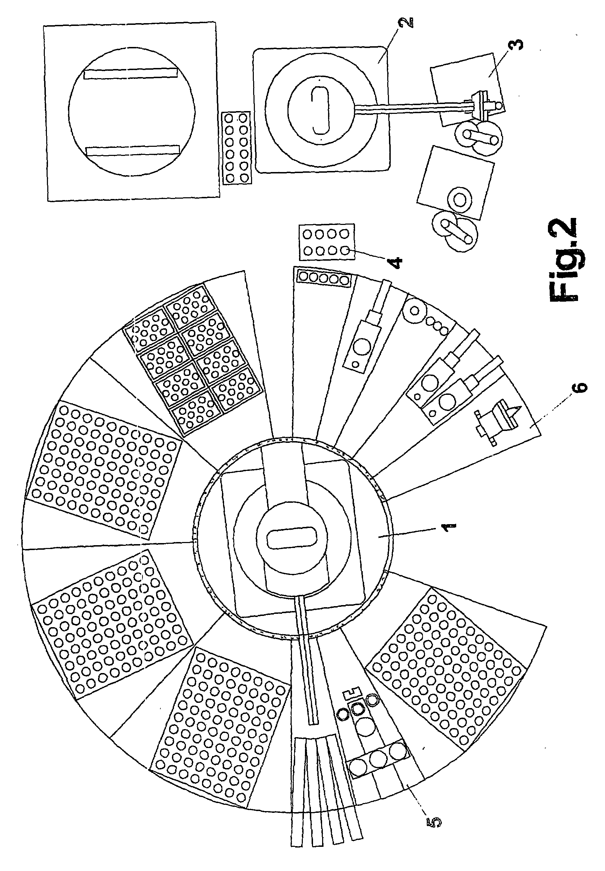

Vorzugsweise erfolgt die Herstellung der erfindungsgemäß zum Einsatz kommenden substituierten γ-Lactonverbindungen auf einer automatischen Anlage der Firma Zymark gemäß **Figur 1** und **Figur 2** wie untenstehend beschrieben.

Anstelle der vorstehend beschriebenen Umsetzung der Reaktionskomponenten der allgemeinen Formeln II und III und der Glyoxalsäure kann auch eine Umsetzung dieser Komponenten ggf. in Gegenwart einer anorganischen und/oder organischen Säure unter Mikrowellenbestrahlung oder unter Einwirkung von Ultraschall erfolgen.

Ebenfalls bevorzugt wird daher zur Herstellung der erfindungsgemäß zum Einsatz kommenden γ-Lactonverbindungen der allgemeinen Formel I, worin die Reste R¹ bis R⁴ die oben angegebene Bedeutung haben, wenigstens eine Aminkomponente der vorstehend angegebenen allgemeinen Formel II, worin R¹ die oben angegebene Bedeutung hat, mit Glyoxalsäure, vorzugsweise in Form des Monohydrats oder einer wäßrigen Lösung, und wenigstens einer Alkenkomponente der oben angegebenen allgemeinen Formel III, worin R² bis R⁴ die oben angegebene Bedeutung haben, in einem organischen Lösungsmittel, ggf. in Gegenwart zumindest einer anorganischen und/oder organischen Säure unter Mikrowellenbestrahlung oder unter Einwirkung von Ultraschall, vorzugsweise unter Mikrowellenbestrahlung, zu wenigstens einer Verbindung der oben angegebenen allgemeinen Formel I umgesetzt und diese gegebenenfalls nach üblichen Methoden gereinigt und/oder gegebenenfalls nach üblichen Methoden isoliert.

Die Mengen der eingesetzten Reaktionskomponenten, die geeignete Temperatur während der Umsetzung und die geeignete Dauer der Umsetzung können variieren. Die für die jeweilige Umsetzung optimale Menge der einzusetzenden Komponenten, die optimale Temperatur sowie die optimale Dauer der Umsetzung können vom Fachman durch einfach Vorversuche ermittelt werden. Sofern die Umsetzung unter Mikrowellenbestrahlung erfolgt, beträgt die Temperatur 40 bis 70 °C, besonders bevorzugt 45 bis 60 °C. Die Dauer der Umsetzung beträgt vorzugsweise 0,1 bis 60 Minuten für die Mikrowellenbestrahlung.

Als geeignetes Lösungsmittel wird bevorzugt Acetonitril oder ein Gemisch enthaltend Acetonitril eingesetzt.

Die jeweiligen Reaktionskomponenten der allgemeinen Formel II und III sowie die Glyoxalsäure können käuflich am Markt erworben oder nach üblichen, dem Fachmann bekannten Methoden hergestellt werden.

Die erfindungsgemäß zum Einsatz kommenden substituierten γ-Lactonverbinclungen der allgemeinen Formel I können nach dem vorstehend beschriebenen Verfahren als freie Base oder als Salz isoliert werden. Die freie Base der jeweiligen Verbindung der allgemeinen Formel I kann nach üblichen, dem Fachmann bekannten Methoden, beispielsweise durch Umsetzung mit einer anorganischen oder organischen Säure, vorzugsweise mit Salzsäure, Bromwasserstoffsäure, Schwefelsäure, Phosphorsäure, Methansulfonsäure, p-Toluolsulfonsäure, Kohlensäure, Ameisensäure, Essigsäure, Oxalsäure, Bernsteinsäure, Weinsäure, Mandelsäure, Fumarsäure, Milchsäure, Citronensäure, Glutaminsäure oder Asparaginsäure, in das entsprechende, physiologisch verträgliche Salz übergeführt werden.
Die freie Base der jeweiligen erfindungsgemäßen Verbindung der allgemeinen Formel I kann auch mit der freien Säure oder einem Salz eines Zuckerersatzstoffes, wie z.B. Saccharin, Cyclamat oder Acesulfam, in das entsprechende physiologisch verträgliche Salz übergeführt werden.

Die Überführung der freien Base der jeweiligen Verbindung der allgemeinen Formel I in das entsprechende Hydrochlorid kann bevorzugt auch durch Versetzen der in einem geeigneten organischen Lösungsmittel, wie z.B. Butan-2-on (Methylethylketon), gelösten Verbindung der allgemeinen Formel I als freie Base mit Trimeahylsilylchlorid (TMSCI) erhalten werden.

Sofern die erfindungsgemäß zum Einsatz kommenden substituierten γ-Lactonverbindungen der allgemeinen Formel I nach dem oben beschriebenen Herstellungsverfahren in Form ihrer Racemate oder anderer Mischungen ihrer verschiedenen Enantiomeren und/oder Diastereomeren erhalten werden, können diese, sofern erforderlich, nach üblichen, dem Fachmann bekannten Verfahren getrennt und ggf. isoliert werden. Beispielhaft seien chromatographische Trennverfahren, insbesondere Flüssigkeitschromatographie-Verfahren unter Normaldruck oder unter erhöhtem Druck, bevorzugt MPLC- und HPLC-Verfahren, sowie Verfahren der fraktionierten Kristallisation genannt. Dabei können insbesondere einzelne Enantiomeren, z.B. mittels HPLC an chiraler Phase oder mittels Kristallisation mit chiralen Säuren, etwa (+)-Weinsäure, (-)-Weinsäure oder (+)-10-Camphersulfonsäure, gebildete diastereomere Salze voneinander getrennt werden.

Die erfindungsgemäß zum Einsatz kommenden γ-Lactonverbindungen der allgemeinen Formel I sind toxikologisch unbedenklich und eignen sich daher als pharmazeutische Wirkstoffe in Arzneimitteln.
Die entsprechenden Arzneimittel können auch Mischungen verschiedener Stereoisomere einer oder mehrerer erfindungsgemäßer γ-Lactonverbindungen enthalten. So können beispielsweise auch verschiedene Enantiomere einer γ-Lactonverbindung der allgemeinen Formel I in nicht äquimolaren Mengen vorliegen.

Die entsprechenden Arzneimittel enthalten neben mindestens einer substituierten γ-Lactonverbindung üblicherweise weitere physiologisch verträgliche Hilfsstoffe, die bevorzugt ausgewählt sind aus der Gruppe bestehend aus Trägermaterialien, Füllstoffen, Lösungsmitteln, Verdünnungsmitteln, Farbstoffen und Bindemitteln. Die entsprechenden Arzneimittel können als flüssige, halbfeste oder feste Arzneiformen, beispielsweise in Form von Injektionslösungen, Tropfen, Säften, Sirupen, Sprays, Suspensionen, Tabletten, Patches, Kapseln, Pflastern, Zäpfchen, Salben, Cremes, Lotionen, Gelen, Emulsionen, Aerosolen oder in multipartikulärer Form, beispielsweise in Form von Pellets oder Granulaten, vorliegen und als solche auch verabreicht werden.

Die Auswahl der physiologisch verträglichen Hilfsstoffe sowie die einzusetzenden Mengen derselben hängt davon ab, ob das jeweilige Arzneimittel oral, peroral, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich beispielsweise Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Substituierte γ-Lactonverbindungen der allgemeinen Formel I in einem Depot in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die substituierten γ-Lactonverbindungen verzögert freisetzen.

Die Herstellung der Arzneimittel erfolgt mit Hilfe von üblichen, dem Fachmann bekannten Mitteln, Vorrichtungen, Methoden und Verfahren, wie sie beispielsweise in "Remington's Pharmaceutical Sciences", Hrsg. A.R. Gennaro, 17. Ed., Mack Publishing Company, Easton, Pa. (1985), insbesondere in Teil 8, Kapitel 76 bis 93, beschrieben sind. Die entsprechende Literaturbeschreibung wird hiermit als Referenz eingeführt und gilt als Teil der Offenbarung.

Die an den Patienten zu verabreichende Menge der jeweiligen substituierten γ-Lactonverbindung der allgemeinen Formel I kann variieren, beispielsweise in Abhängigkeit vom Gewicht oder dem Alter des Patienten sowie von der Applikationsart, der Indikation und dem Schweregrad der Erkrankung. Üblicherweise werden 0,02 bis 500 mg pro kg, vorzugsweise 0,2 bis 5 mg, Körpergewicht des Patienten wenigstens einer substituierten γ-Lactonverbindung der allgemeinen Formel I appliziert.

### Molekularpharmakologische Untersuchungen:

Im folgenden werden die zur Bestimmung der Stickstoffmonoxid-Synthase Inhibierung durch die erfindungsgemäß zum Einsatz kommenden Verbindungen der allgemeinen Formel I verwendeten Assays beschrieben:

### Stickstoffmonoxid-Synthase (NOS)-Assay

Dieser Assay erlaubt die Bestimmung der prozentualen Hemmung von NO-Synthase durch eine erfindungsgemäß zum Einsatz kommende Verbindung der allgemeinen Formel I mittels Messung der NOS-Aktivität bei Einwirken der Verbindung. Dabei wird NO-Synthase zusammen mit radioaktiv markiertem Arginin und der jeweiligen Verbindung der allgemeinen Formel I unter geeigneten Bedingungen gemischt. Nach Abbruch der NO-Bildungsreaktion zu einem vorgegebenen Zeitpunkt wird die Menge an nicht umgesetztem Arginin direkt oder indirekt bestimmt. Der Vergleich dieser Menge mit der in einem ohne Zusatz einer Verbindung der allgemeinen Formel I und unter sonst gleichen Bedingungen aus der Mischung von NOS und Arginin zurückbleibenden Menge an Arginin ergibt die prozentuale Hemmung von NO-Synthase durch die getetstete Verbindung. Dieser Assay läßt sich wie folgt durchführen:
(a) Inkubation der NO-Synthase mit markiertem Arginin als Substrat in einem Reaktionsgefäß,
(b) Trennung des markierten Arginins von dem gegebenenfalls als Produkt der enzymatischen Reaktion entstandenen, markierten Citrullin zu einem Zeitpunkt, zu dem die Konzentration an Citrullin ansteigt,
(c) Messung der Menge an jeweils abgetrenntem Arginin.

Die Trennung erfolgt über eine Filterplatten-Membran.

Dieser NOS-Assay eignet sich insbesondere für ein "High Throughput Screening" (HTS) auf Mikrotiterplatten (MTP).

### HTS-NOS-Assay: Allgemeine Verfahrensweise

In diesem HTS-NOS-Assay wird radioaktives Arginin als Substrat benutzt. Das Assayvolumen kann je nach Art der Mikrotiterplatte (MTP) im Bereich zwischen 25 µl und 250 µl gewählt werden. In Abhängigkeit von der benutzten Enzymquelle werden Cofaktoren und Coenzyme zugefügt. Die Inkubation der Ansätze in dieser Mikrotiterplatte (Assay-MTP) gemäß Schritt (a) wird bei Raumtemperatur vorgenommen und beträgt je nach verwendeter Enzymaktivität (units) zwischen 5 und 60 Minuten. Zum Ende der Inkubation (Schritt (a)) wird die Platte in einen Zellharvester plaziert, der mit einer MTP bestückt ist, die eine Kationenaustauschermembran als Filterboden besitzt (Filter-MTP). Alle Ansätze der Assay-MTP werden in diese Filter-MTP überführt und über eine Kationenaustauscher-Filter-Platte, einen mit Phosphatgruppen beladenen Papierfilter, abgesaugt. Die Filter-MTP wird anschließend mit Puffer oder Wasser gewaschen. Mit Hilfe dieser Vorgehensweise wird das verbliebene Substrat Arginin auf dem Kationenaustauscher gebunden, während das enzymatisch gebildete radioaktive Citrullin quantitativ ausgewaschen wird. Nach Trocknen der Filter-MTP und Zugabe von Szintillationsflüssigkeit kann das gebundene Arginin am Szintillationszähler ausgezählt werden. Eine nicht gehemmte NOS-Reaktion spiegelt sich in einer geringen Radioaktivität wieder. Eine gehemmte Enzymreaktion bedeutet, daß das radioaktive Arginin nicht umgesetzt worden ist. Das heißt, auf dem Filter befindet sich eine hohe Radioaktivität.

### Verwendete Materialien

- Arginin, L-[2, 3, 4-³H]-monohydrochlorid; Best.-Nr. NET-1123, Firma NEN
- CaCl₂ wasserfrei; Best.- Nr. 2388.1000; Firma Merck KGaA
- 1.4-Dithiothreitol (DTT), Best.-Nr. 708984; Firma ROCHE
- Na₂EDTA-Dihydrat; Best.-Nr. 03680; Firma FLUKA
- HEPES, Best:-Nr. H-3375; Firma SIGMA
- NADPH, Tetranatriumsalz; Best.-Nr. 1585363; Firma ROCHE
- TRIS; BEST.-Nr. 93349; Firma FLUKA

| | |
|---|---|
| Enzym-Präparationspuffer | 50 mM Tris-HCl mit 1 mM EDTA: Der pH-Wert des Puffers wurde bei 4 °C auf 7,4 eingestellt. |
| | |
| Inkubationspuffer (-medium) | 50 mM HEPES mit 1 mM EDTA; 1,25 mM CaCl₂ und 1 mM Dithiothreitol. Der pH-Wert des Puffers wurde bei 25 °C auf 7,4 eingestellt. |
| | |
| Waschmedium | H₂O |

### Enzympräparation

Als Ausgangsgewebe wurden Ratten-Cerebelli benutzt. Die Tiere wurden betäubt und getötet, das Gehirngewebe, das Cerebellum, wurde herauspräpariert, pro Rattenkleinhirn wurde 1 ml Enzympräparationspuffer (4 °C) hinzugegeben, und es wurde mit einem Polytron-Homogenisierer für 1 min bei 6000 U/min aufgeschlossen. Danach erfolgte Zentrifugation bei 4°C für 15 min bei 20 000 g und anschließend Abdekantieren des Überstand und portioniertes Einfrieren bei -80 °C (Verwerfen des Niederschlags).

### Inkubationsansatz:

Verwendet wurden 96-well MTP mit einer "Well"-Kapazität von ≤ 250 µl Pipettierreihenfolge: siehe Tabelle 1:

**Tabelle 1:**

| **Substanz** | **Molarität i.A.** | **µl** | *** Protein i.A:** |
|---|---|---|---|
| Inkubations-Puffer | - | 100 | - |
| Testsubstanz | variabel; vorzugsweise 10⁻⁵M | variabel; vorzugsweise 20 µl | - |
| NADPH | 0,5 mM | 20 | - |
| Enzym | - | variabel; maximales Volumen der Enzymlösung = 50 µl | variabel; maximale einsetzbare Proteinmenge = 100 µg |
| [³H]Substrat | variabel; vorzugsweise 50 nM | variabel; vorzugsweise 10 µl | - |
| Endvolumen: | | max. 250 µl | |

Die Proteinbestimmung erfolgte nach O.H. Lowry et al; J. Biol.Chem. 193, 265 (1951). Die entsprechende Literaturbeschreibung wird hiermit als Referenz eingeführt und gilt als Teil der Offenbarung.
i.A. = im Ansatz

Nach beendetem Pipettiervorgang wurde ein Deckel auf diese MTP (Assay-MTP) gelegt. Inkubation bei 25 °C (Raumtemperatur (RT)) für 5-60 min, je nach Menge und Aktivität des eingesetzten Enzyms.

Anschließend wurde der Inhalt der Assay-MTP mit Hilfe eines 96-well Cell-Harvesters in eine 96-well Kationenaustauscher MTP (Filter-MTP) transferiert und abgesaugt. Es schloß sich eine einmalige Wäsche mit 200 ml H₂O (aus einer Wanne) an.

Dann wurde die Platte für 1 h bei 60 C im Trockenschrank getrocknet. Dann wurde die Bodenseite der Filter-MTP von unten her exakt mit einem "back seal" versiegelt. Danach wurden pro well 35 µl Szintillator hinzupipettiert. Ferner wurde die Plattenoberseite mit einem "top seal" versiegelt. Nach 1 h Wartezeit wurde die Platte am β-Counter ausgemessen.

Im HTS-Betrieb wurden das Inkubationsmedium, NADPH- und Enzymlösung vor Beginn des Pipettierschrittes vereint, um nicht zeitaufwendig drei separate Pipettierungen vornehmen zu müssen.

Im folgenden wird die Erfindung anhand von Beispielen erläutert. Diese Erläuterungen sind lediglich beispielhaft und schränken den allgemeinen Erfindungsgedanken nicht ein.

### Beispiele:

Die zur Herstellung der γ-Lactonverbindungen der allgemeinen Formel I eingesetzten Chemikalien und Lösungsmittel wurden kommerziell, beispielsweise von Acros, Avocado, Aldrich, Fluka, Lancaster, Maybridge, Merck, Sigma oder TCl, erworben oder nach üblichen, dem Fachmann bekannten Methoden hergestellt.

Die dünschichtchromatographischen Untersuchungen wurden mit HPTLC-Fertigplatten, Kieselgel 60 F 254, der Firma E. Merck, Darmstadt, durchgeführt.

Die Ausbeuten der hergestellten Verbindungen wurden nicht optimiert.

Die Analytik erfolgte über ESI-Massenspektroskopie oder NMR-Spektroskopie.

### Allgemeine Arbeitsvorschrift 1:

Die Synthese der γ-Lactonverbindungen der allgemeinen Formel I erfolgte auf einer automatischen Anlage der Firma Zymark gemäß Figur 1 und Figur 2.

Dabei umfaßt Figur 1 eine Capper-Station (Ziff. 1) zum Verschließen der Reaktionsröhrchen, einen Roboter 1 (Ziff. 2) und einen Roboter 2 (Ziff. 3), wobei der Roboter 1 die Reaktionsröhrchen bzw. die entsprechenden Racks bewegt und der Roboter 2 die Reagenzien in die Reaktionsröhrchen pipettiert, einen temperierbaren Reaktorblock (Ziff. 4), Rührblöcke (Ziff. 5) und eine Filtrationsstation (Ziff. 6), in der die Reaktionslösung abfiltriert wird.

Figur 2 umfaßt ebenfalls einen Roboter 1 (Ziff. 1) und einen Roboter 2 (Ziff. 2), die beide die Glasröhrchen mit den Syntheseprodukten auf die verschiedenen Stationen bewegen. Bei den Stationen handelt es sich im einzelnen um einen Vortexer (Ziff. 3) zum Durchmischen der Proben und zum Zudosieren von Lösungen oder Lösungsmitteln, einen Spin-Reaktor (Ziff. 4) zur Durchmischung von Proben, eine Phasendetektionsstation (Ziff. 5) zur Detektion der Phasengrenze und Phasentrennung sowie eine Station (Ziff. 6) zum Trocknen der Syntheseprodukte über Salzkartuschen.

Zur Synthese wurde ein Rundbodenröhrchen aus Glas (Durchmesser 16 mm, Länge 125 mm) mit Gewinde manuell mit einem Rührer versehen und auf der Capper-Station (Ziff. 1) gemäß Figur 1 mit einem Schraubdeckel mit Septum verschlossen. Das Röhrchen wurde von Roboter 1 (Ziff. 2) in den auf 20 °C temperierten Reaktorblock (Ziff. 4) gestellt. Roboter 2 (Ziff. 3) pipettierte nacheinander folgende Reagenzien hinzu:
1.) 1 ml einer Lösung aus Trifluoressigsäure und der jeweiligen Aminkomponente, jeweils 0,1 M in Acetonitril,
2.) 1 ml einer 0,11 M Glyoxalsäure-Monohydrat-Lösung in Acetonitril
3.) 1 ml einer 0,3 M Lösung der jeweiligen Alkenkomponente in Acetonitril

Das Reaktionsgemisch wurde im Anschluss bei 20 °C in einem der Rührblöcke (Ziff. 5) 600 min lang gerührt. Danach wurde die Reaktionslösung an der Filtrations-Station (Ziff. 6) abfiltriert. Das Röhrchen wurde dabei zweimal mit je 1,5 ml einer 7,5 Gew.-% Natriumhydrogencarbonat-Lösung gespült.

Das Rack mit den Röhrchen wurde anschließend manuell auf eine automatische Aufarbeitungsanlage gemäß Figur 2 gestellt. Dort wurde das Reaktionsgernisch auf einem Vortexer (Ziff. 3) mit 2 ml Diethylether versetzt und geschüttelt.

Im Spin-Reaktor (Ziff. 4) wurde zehn Minuten lang gründlich gemischt und durch die langsame Abnahme der Drehbewegung eine deutliche Phasengrenze ausgebildet. Diese Phasengrenze wurde auf der Phasendetektionsstation (Ziff. 5) optisch detektiert und die organische Phase abpipettiert. Im nächsten Schritt wurde die wäßrige Phase erneut mit 2 ml Diethylether versetzt, geschüttelt, zentrifugiert und die organische Phase abpipettiert. Die vereinigten organischen Phasen wurden über 2,4 g MgSO₄ (granuliert) getrocknet. Das Lösungsmittel wurde in einer Vakuumzentrifuge entfernt. Jede Probe wurde anschließend mit Elektronenspray-Ionisations-Massenspektrometrie (ESI-MS) und/oder NMR-Spektroskopie analysiert.

Durch die automatisierte Synthese ist eine Gleichbehandlung aller Proben sowie eine konstante Reaktionsführung gewährleistet. Die nach der vorstehenden allgemeinen Arbeitsvorschrift hergestellten beispielgemäßen γ-Lactonverbindungen sind in der nachfolgenden Tabelle 2 angegeben:

**Tabelle 2:**

| **Beispiel** | **Bezeichnung der Verbindung:** |
|---|---|
| 1 | 5-(2,4-Dimethyl-phenyl)-3-(8-hydroxy-chinolin-2-ylamino)-5-methyl-dihydro-furan-2-on |
| 2 | 5-(3,4-Dimethyl-phenyl)-3-(4,6-dimethyl-pyridin-2-ylamino)-5-methyl-dihydro-furan-2-on |
| 3 | 5-(2,4-Dimethyl-phenyl)-3-(4,6-dimethyl-pyridin-2-ylamino)-5-methyl-dihydro-furan-2-on |
| 4 | 5-(4-Cyclohexyl-phenyl)-3-(4,6-dimethyl-pyridin-2-ylamino)-5-methyl-dihydro-furan-2-on |
| 5 | 5-(3,5-Dimethyl-phenyl)-3-(4,6-dimethyl-pyridin-2-ylamino)-5-methyl-dihydro-furan-2-on |
| 6 | 5-(3,4-Dimethyl-phenyl)-5-methyl-3-(4-methyl-pyridin-2-ylamino)-dihydro-furan-2-on |
| 7 | 5-(2,4-Dimethyl-phenyl)-5-methyl-3-(4-methyl-pyridin-2-ylamino)-dihydro-furan-2-on |
| 8 | 5-(4-Cyclohexyl-phenyl)-5-methyl-3-(4-methyl-pyridin-2-ylamino)-dihydro-furan-2-on |
| 9 | 5-Methyl-3-(chinolin-2-ylamino)-5-m-tolyl-dihydro-furan-2-on |
| 10 | 3-(4,6-Dimethyl-pyridin-2-ylamino)-5-methyl-5-p-tolyl-dihydro-furan-2-on |
| 11 | 3-(4,6-Dimethyl-pyridin-2-ylamino)-5-methyl-5-m-tolyl-dihydrofuran-2-on |
| 12 | 3-(4,6-Dimethyl-pyridin-2-ylamino)-5-(4-ethoxy-phenyl)-5-methyldihydro-furan-2-on |
| 13 | 4-[4-(3-Brom-5-methyl-pyridin-2-ylamino)-2-methyl-5-oxo-tetrahydro-furan-2-yl]-benzonitril |
| 14 | 3-(3-Brom-5-methyl-pyridin-2-ylamino)-5-(4-tert-butyl-phenyl)-5-methyl-dihydro-furan-2-on |
| 15 | 5-(4-tert-Butyl-phenyl)-5-methyl-3-(6-propyl-pyridin-2-ylamino)-dihydro-furan-2-on |
| 16 | 5-(4-tert-Butyl-phenyl)-5-methyl-3-(4-methyl-3-nitro-pyridin-2-ylamino)-dihydro-furan-2-on |
| 17 | 3-(5-Brom-6-methyl-pyridin-2-ylamino)-5-methyl-5-(4-phenoxyphenyl)-dihydro-furan-2-on |
| 18 | 5-(4-tert-Butyl-phenyl)-5-methyl-3-(3-nitro-pyridin-2-ylamino)-dihydro-furan-2-on |
| 19 | 3-(3-Benzyloxy-pyridin-2-ylamino-5-methyl-5-(4-phenoxy-phenyl)-dihydro-furan-2-on |
| 20 | 3-(3-Benzyloxy-pyridin-2-ylamino)-5-(4-tert-butyl-phenyl)-5-methyl-dihydro-furan-2-on |
| 21 | 3-(4,6-Dimethyl-pyridin-2-ylamino)-5-methyl-5-(4-phenoxyphenyl)-dihydro-furan-2-on |
| 22 | 5-(4-tert-Butyl-phenyl)-3-(4,6-dimethyl-pyridin-2-yl-amino)-5-methyl dihydrofuran-2-on |
| 23 | 5-Methyl-3-(4-methyl-pyridin-2-ylamino)-5-(4-phenoxy-phenyl)-dihydro-furan-2-on |
| 24 | 5-(4-tert-Butyl-phenyl)-5-methyl-3-(4-methyl-pyridin-2-ylamino)-dihydro-furan-2-on |
| 25 | 4-[4-(5-Brom-3-nitro-pyridin-2-ylamino)-2-methyl-5-oxo-tetrahydro-furan-2-yl]-benzonitril |
| 26 | 4-[4-(5-Brom-pyrimidin-2-ylamino)-2-methyl-5-oxo-tetrahydro-furan-2-yl]-benzonitril |
| 27 | 5-Benzo[b]thiophen-2-yl-5-methyl-3-(6-propyl-pyridin-2-ylamino)-dihydro-furan-2-on |
| 28 | 3-(4,6-Dimethyl-pyridin-2-ylamino)-5-(4-isopropyl-phenyl)-5-methyl-dihydro-furan-2-on |
| 29 | 5-Benzofuran-2-yl-3-(4,6-dimethyl-pyridin-2-ylamino)-5-methyl-dihydro-furan-2-on |
| 30 | 5-Benzo[b]thiophen-2-yl-5-methyl-3-(4-methyl-pyridin-2-ylamino)-dihydro-furan-2-on |
| 31 | 5-Benzofuran-2-yl-5-methyl-3-(4-methyl-pyridin-2-ylamino)-dihydro-furan-2-on |
| 32 | 3-(5-Benzo[1,3]dioxol-5-yl-5-methyl-2-oxo-tetrahydro-furan-3-ylamino)-1H-pyrazol-4-carbonitril |
| 33 | 3-(5-Benzo[1,3]dioxol-5-yl-5-methyl-2-oxo-tetrahydro-furan-3-ylamino)-1H-pyrazol-4-carbonsäureethylester |
| 34 | 5-Benzo[1,3]dioxol-5-yl-5-methyl-3-(3-nitro-pyridin-2-ylamino)-dihydro-furan-2-on |
| 35 | 3-(4,6-Dimethyl-pyridin-2-ylamino)-5-methyl-5-(5,6,7,8-tetrahydro-naphthalin-2-yl)-dihydro-furan-2-on |
| 36 | 3-(4,6-Dimethyl-pyridin-2-ylamino)-5-methyl-5-naphthalin-2-yl-dihydro-furan-2-on |
| 37 | 5-Benzo[1,3]dioxol-5-yl-5-methyl-3-(4-methyl-pyridin-2-ylamino)-dihydro-furan-2-on |
| 38 | 5-Methyl-3-(4-methyl-pyridin-2-ylamino)-5-(5,6,7,8-tetrahydro-naphthalin-2-yl)-dihydro-furan-2-on |
| 39 | 5-Benzo[1,3]dioxol-5-yl-5-methyl-3-(5-methyl-pyridin-2-ylamino)-dihydro-furan-2-on |
| 40 | 5-Benzo[1,3]dioxol-5-yl-5-methyl-3-(6-methyl-pyridin-2-ylamino)-dihydro-furan-2-on |
| 41 | 3-(5-Brom-3-nitro-pyridin-2-ylamino)-5-methyl-5-(5,6,7,8-tetrahydro-naphthalin-2-yl)-dihydro-furan-2-on |
| 42 | 3-(5-Brom-3-nitro-pyridin-2-ylamino)-5-isopropyl-5-phenyl-dihydro-furan-2-on |
| 43 | 5-Isopropyl-3-(5-nitro-pyridin-2-ylamino)-5-phenyl-dihydro-furan-2-on |
| 44 | 5-Methyl-5-naphthalin-2-yl-3-(5-nitro-pyridin-2-ylamino)-dihydro-furan-2-on |
| 45 | 5-Isopropyl-5-phenyl-3-(pyrimidin-2-ylamino)-dihydro-furan-2-on |
| 46 | 3-[5-(4-Iod-phenyl)-5-methyl-2-oxo-tetrahydro-furan-3-ylamino]-1H-pyrazol-4-carbonsäureethylester |
| 47 | 5-(4-Brom-phenyl)-3-(5-brom-pyridin-2-ylamino)-5-methyl-dihydro-furan-2-on |
| 48 | 3-(3-Brom-5-methyl-pyridin-2-ylamino)-5-methyl-5-naphthalin-1-yl-dihydro-furan-2-on |
| 49 | 5-Methyl-5-naphthalin-1-yl-3-(6-propyl-pyridin-2-ylamino)-dihydro-furan-2-on |
| 50 | 5-(3-Chlor-phenyl)-5-methyl-3-(6-propyl-pyridin-2-ylamino)-dihydro-furan-2-on |
| 51 | 5-(3-Chlor-phenyl)-5-methyl-3-(4-methyl-3-nitro-pyridin-2-ylamino)-dihydro-furan-2-on |
| 52 | 5-(4-Brom-phenyl)-5-methyl-3-(4-methyl-3-nitro-pyridin-2-ylamino)-dihydro-furan-2-on |
| 53 | 3-(5-Brom-6-methyl-pyridin-2-ylamino)-5-methyl-5-naphthalin-1-yl-dihydro-furan-2-on |
| 54 | 3-(5-Brom-6-methyl-pyridin-2-ylamino)-5-(4-iodo-phenyl)-5-methyl-dihydro-furan-2-on |
| 55 | 3-(3-Benzyloxy-pyridin-2-ylamino)-5-(4-iodo-phenyl)-5-methyl-dihydro-furan-2-on |
| 56 | 3-(3-Benzyloxy-pyridin-2-ylamino)-5-(4-brom-phenyl)-5-methyl-dihydro-furan-2-on |
| 57 | 3-(4,6-Dimethyl-pyridin-2-ylamino)-5-(4-iodo-phenyl)-5-methyl-dihydro-furan-2-on |
| 58 | 5-(3-Chlor-phenyl)-3-(4,6-dimethyl-pyridin-2-ylamino)-5-methyl-dihydro-furan-2-on |
| 59 | 5-(4-Brom-phenyl)-5-methyl-3-(3-methyl-pyridin-2-ylamino)-dihydro-furan-2-on |
| 60 | 5-(4-Brom-phenyl)-5-methyl-3-(4-methyl-pyridin-2-ylamino)-dihydro-furan-2-on |
| 61 | 2-[5-(3,5-Dimethoxy-phenyl)-5-methyl-2-oxo-tetrahydro-furan-3-ylamino]-4-propyl-pyrimidin-5-carbonsäureethylester |
| 62 | 3-(4-Brom-1H-pyrazol-3-ylamino)-5-(3,5-dimethoxy-phenyl)-5-methyl-dihydro-furan-2-on |
| 63 | 3-(4-Brom-1H-pyrazol-3-ylamino)-5-(2-methoxy-phenyl)-5-methyl-dihydro-furan-2-on |
| 64 | 3-[5-(2,5-Dimethoxy-phenyl)-5-methyl-2-oxo-tetrahydro-furan-3-ylamino]-1H-pyrazol-4-carbonitril |
| 65 | 3-[5-(2,5-Dimethoxy-phenyl)-5-methyl-2-oxo-tetrahydro-furan-3-ylamino]-5-methylsulfanyl-1H-pyrazol-4-carbonitril |
| 66 | 5-(2,5-Dimethoxy-phenyl)-5-methyl-3-(pyridin-2-ylamino)-dihydro-furan-2-on |
| 67 | 5-(2-Methoxy-phenyl)-5-methyl-3-(pyridin-2-ylamino)-dihydro-furan-2-on |
| 68 | 3-(3-Chlor-5-triffuormethyl-pyridin-2-ylamino)-5-(3,5-dimethoxy-phenyl)-5-methyl-dihydro-furan-2-on |
| 69 | 3-(3-Chlor-5-trifluormethyl-pyridin-2-ylamino)-5-(2,5-dimethoxy-phenyl)-5-methyl-dihydro-furan-2-on |
| 70 | 3-(3,5-Dichlor-pyridin-2-ylamino)-5-(2-methoxy-phenyl)-5-methyl-dihydro-furan-2-on |
| 71 | 3-(3-Chlor-5-trifluormethyl-pyridin-2-ylamino)-5-(2,4-dimethoxy-phenyl)-5-methyl-dihydro-furan-2-on |
| 72 | 3-(4,6-Dimethyl-pyridin-2-ylamino)-5-(3-methoxy-phenyl)-5-methyl-dihydro-furan-2-on |
| 73 | 3-(4,6-Dimethyl-pyridin-2-ylamino)-5-(4-methoxy-phenyl)-5-methyl-dihydro-furan-2-on |
| 74 | 5-(3,4-Dimethoxy-phenyl)-3-(4,6-dimethyl-pyridin-2-ylamino)-5-methyl-dihydro-furan-2-on |
| 75 | 5-(4-Methoxy-phenyl)-5-methyl-3-(4-methyl-pyridin-2-ylamino)-dihydro-furan-2-on |
| 76 | 5-(2,5-Dimethoxy-phenyl)-5-methyl-3-(pyrazin-2-ylamino)-dihydro-furan-2-on |
| 77 | 5-Methylsulfanyl-3-(2-oxo-5-phenyl-5-propyl-tetrahydro-furan-3-ylamino)-1H-pyrazol-4-carbonitril |

### Molekularpharmakologische Untersuchung:

Die gemäß den Beispielen 1 bis 75 hergestellten γ-Lactonverbindungen wurden, wie obenstehend beschrieben, im HTS-NOS-Assay getestet. Die mit einer Messung festgestellte Hemmung der Stickstoffmonoxid-Synthase durch die beispielgemäßen Verbindungen (10 µM) ist in der nachfolgenden Tabelle 3 wiedergegeben:

**Tabelle 3:**

| **Beispiel- Nr.** | **NOS** **(%Hemmung)** |
|---|---|
| 1 | 40 |
| 2 | 45 |
| 3 | 42 |
| 4 | 45 |
| 5 | 58 |
| 6 | 40 |
| 7 | 45 |
| 8 | 41 |
| 9 | 51 |
| 10 | 61 |
| 11 | 81 |
| 12 | 59 |
| 13 | 44 |
| 14 | 48 |
| 15 | 45 |
| 16 | 47 |
| 17 | 43 |
| 18 | 40 |
| 19 | 40 |
| 20 | 54 |
| 21 | 91 |
| 22 | 44 |
| 23 | 79 |
| 24 | 84 |
| 25 | 45 |
| 26 | 43 |
| 27 | 43 |
| 28 | 68 |
| 29 | 40 |
| 30 | 41 |
| 31 | 44 |
| 32 | 41 |
| 33 | 45 |
| 34 | 40 |
| 35 | 42 |
| 36 | 65 |
| 37 | 50 |
| 38 | 55 |
| 39 | 44 |
| 40 | 53 |
| 41 | 53 |
| 42 | 41 |
| 43 | 45 |
| 44 | 47 |
| 45 | 58 |
| 46 | 44 |
| 47 | 54 |
| 48 | 45 |
| 49 | 53 |
| 50 | 61 |
| 51 | 50 |
| 52 | 43 |
| 53 | 40 |
| 54 | 70 |
| 55 | 44 |
| 56 | 59 |
| 57 | 72 |
| 58 | 73 |
| 59 | 45 |
| 60 | 59 |
| 61 | 40 |
| 62 | 40 |
| 63 | 51 |
| 64 | 42 |
| 65 | 42 |
| 66 | 52 |
| 67 | 45 |
| 68 | 60 |
| 69 | 58 |
| 70 | 51 |
| 71 | 54 |
| 72 | 40 |
| 73 | 56 |
| 74 | 51 |
| 75 | 48 |

Alle untersuchten beispielgemäßen γ-Lactonverbindungen zeigen eine gute Hemmung der Stickstoffmonoxid-Synthase.

## Patentansprüche

1. Verwendung wenigstens einer substituierten γ-Lactonverbindung der allgemeinen Formel I, 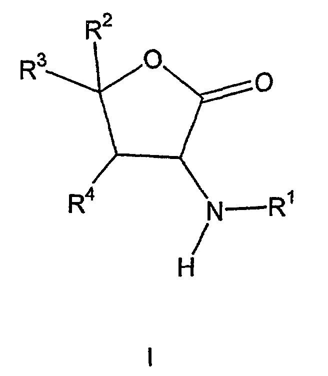 worin
R¹ für einen gegebenenfalls wenigstens einfach substituierten 2-Pyridyl-, 2-Pyrimidyl-, 2-Pyrazolyl-, 2-Chinolinyl- oder 2-Pyrazinyl-Rest steht, der auch mit einem gesättigten oder zumindest teilweise ungesättigten Kohlenwasserstoff-Ringsystem kondensiert sein kann,
R² für einen gegebenenfalls wenigstens einfach substituierten, gesättigten, verzweigten oder unverzweigten aliphatischen C₁₋₁₀-Rest oder einen gegebenenfalls wenigstens einfach substituierten, zumindest teilweise ungesättigten, verzweigten oder unverzweigten aliphatischen C₂₋₁₀-Rest steht,
R³ für einen gegebenenfalls wenigstens einfach substituierten Aryl-Rest steht,
R⁴ für H steht,
oder
R³ und R⁴ zusammen für einen gegebenenfalls wenigstens einfach substituierten, gesättigten oder wenigstens einfach ungesättigten aliphatischen C₃₋₇-Rest stehen, mit der Maßgabe, daß der Rest R² in diesem Fall für einen gegebenenfalls wenigstens einfach substituierten Aryl-Rest, für einen gegebenenfalls wenigstens einfach substituierten, gesättigten, verzweigten oder unverzweigten aliphatischen C₁₋₁₀-Rest oder für einen gegebenenfalls wenigstens einfach substituierten, zumindest teilweise ungesättigten, verzweigten oder unverzweigten aliphatischen C₂₋₁₀-Rest steht,
in Form ihrer Racemate, Diastereomere oder Enantiomere als freie Base oder eines entsprechenden physiologisch verträglichen Salzes zur Herstellung eines Arzneimittels zur Behandlung von Migräne.

2. Verwendung gemäß Anspruch 1, **dadurch gekennzeichnet, daß** R¹ für einen gegebenenfalls wenigstens einfach substituierten 2-Pyridyl-Rest steht, der auch mit einem gesättigten oder zumindest teilweise ungesättigten Kohlenwasserstoff-Ringsystem kondensiert sein kann, vorzugsweise für einen wenigstens in 4-Stellung substituierten 2-Pyridyl-Rest steht.

3. Verwendung gemäß Anspruch 1 oder 2, **dadurch gekennzeichnet, daß** R² für einen gegebenenfalls wenigstens einfach substituierten, verzweigten oder unverzweigten C₁₋₆-Rest, steht.

4. Verwendung gemäß einem der Ansprüche 1 bis 3, **dadurch gekennzeichnet, daß** R³ für einen gegebenenfalls wenigstens einfach substituierten Aryl-Rest und R⁴ für H steht.

5. Verwendung gemäß einem der Ansprüche 1 bis 4, **dadurch gekennzeichnet, daß** als Verbindung der allgemeinen Formel I gemäß Anspruch 1 wenigstens eine Verbindung ausgewählt aus der Gruppe bestehend aus
5-(2,4-Dimethyl-phenyl)-3-(8-hydroxy-chinolin-2-ylamino)-5-methyl-dihydrofuran-2-on,
5-(3,4-Dimethyl-phenyl)-3-(4,6-dimethyl-pyridin-2-ylamino)-5-methyldihydro-furan-2-on,
5-(2,4-Dimethyl-phenyl)-3-{4,6-dimethyl-pyridin-2-ylamino)-5-methyldihydro-furan-2-on,
5-(4-Cyclohexyl-phenyl)-3-(4,6-dimethyl-pyridin-2-ylamino)-5-methyldihydro-furan-2-on,
5-(3,5-Dimethyl-phenyl)-3-(4,6-dimethyl-pyridin-2-ylamino)-5-methyldihydro-furan-2-on,
5-(3,4-Dimethyl-phenyl)-5-methyl-3-(4-methyl-pyridin-2-ylamino)-dihydrofuran-2-on,
5-(2,4-Dimethyl-phenyl)-5-methyl-3-(4-methyl-pyridin-2-ylamino)-dihydrofuran-2-on,
5-(4-Cyclohexyl-phenyl)-5-methyl-3-(4-methyl-pyridin-2-ylamino)-dihydrofuran-2-on,
5-Methyl-3-(chinolin-2-ylamino)-5-m-tolyl-dihydro-furan-2-on,
3-(4,6-Dimethyl-pyridin-2-ylamino)-5-methyl-5-p-tolyl-dihydro-furan-2-on,
3-(4,6-Dimethyl-pyridin-2-ylamino)-5-methyl-5-m-tolyl-dihydro-furan-2-on,
3-(4,6-Dimethyl-pyridin-2-ylamino)-5-(4-ethoxy-phenyl)-5-methyl-dihydrofuran-2-on,
4-[4-(3-Brom-5-methyl-pyridin-2-ylamino)-2-methyl-5-oxo-tetrahydro-furan-2-yl]-benzonitril,
3-(3-Brom-5-methyl-pyridin-2-ylamino)-5-(4-tert-butyl-phenyl)-5-methyldihydro-furan-2-on,
5-(4-tert-Butyl-phenyl)-5-methyl-3-(6-propyl-pyridin-2-ylamino)-dihydrofuran-2-on,
5-(4-tert-Butyl-phenyl)-5-methyl-3-(4-methyl-3-nitro-pyridin-2-ylamino)-dihydro-furan-2-on,
3-(5-Brom-6-methyl-pyridin-2-ylamino)-5-methyl-5-(4-phenoxy-phenyl)-dihydro-furan-2-on,
5-(4-tert-Butyl-phenyl)-5-methyl-3-(3-nitro-pyridin-2-ylamino)-dihydro-furan-2-on,
3-(3-Benzyloxy-pyridin-2-ylamino)-5-methyl-5-(4-phenoxy-phenyl)-dihydrofuran-2-on,
3-(3-Benzyloxy-pyridin-2-ylamino)-5-(4-tert-butyl-phenyl)-5-methyl-dihydrofuran-2-on,
3-(4,6-Dimethyl-pyridin-2-ylamino)-5-methyl-5-(4-phenoxy-phenyl)-dihydrofuran-2-on,
5-(4-tert-Butyl-phenyl)-3-(4,6-dimethyl-pyridin-2-yl-amino)-5-methyldihydrofuran-2-on,
5-Methyl-3-(4-methyl-pyridin-2-ylamino)-5-(4-phenoxy-phenyl)-dihydrofuran-2-on,
5-(4-tert-Butyl-phenyl)-5-methyl-3-(4-methyl-pyridin-2-ylamino)-dihydrofuran-2-on,
4-[4-(5-Brom-3-nitro-pyridin-2-ylamino)-2-methyl-5-oxo-tetrahydro-furan-2-yl]-benzonitril,
4-[4-(5-Brom-pyrimidin-2-ylamino)-2-methyl-5-oxo-tetrahydro-furan-2-yl]-benzonitril,
5-Benzo[b]thiophen-2-yl-5-methyl-3-(6-propyl-pyridin-2-ylamino)-dihydrofuran-2-on,
3-(4,6-Dimethyl-pyridin-2-ylamino)-5-(4-isopropyl-phenyl)-5-methyl-dihydrofuran-2-on,
5-Benzofuran-2-yl-3-(4,6-dimethyl-pyridin-2-ylamino)-5-methyl-dihydrofuran-2-on,
5-Benzo[b]thiophen-2-yl-5-methyl-3-(4-methyl-pyridin-2-ylamino)-dihydrofuran-2-on,
5-Benzofuran-2-yl-5-methyl-3-(4-methyl-pyridin-2-ylamino)-dihydro-furan-2-on,
3-(5-Benzo[1,3]dioxol-5-yl-5-methyl-2-oxo-tetrahydro-furan-3-ylamino)-1Hpyrazol-4-carbonitril,
3-(5-Benzo[1,3]dioxol-5-yl-5-methyl-2-oxo-tetrahydro-furan-3-ylamino)-1H-pyrazol-4-carbonsäureethylester,
5-Benzo[1,3]dioxol-5-yl-5-methyl-3-(3-nitro-pyridin-2-ylamino)-dihydro-furan-2-on,
3-(4,6-Dimethyl-pyridin-2-ylamino)-5-methyl-5-(5,6,7,8 tetrahydronaphthalin-2-yl)-dihydro-furan-2-on,
3-(4,6-Dimethyl-pyridin-2-ylamino)-5-methyl-5-naphthalin-2-yl-dihydro-furan-2-on,
5-Benzo[1,3]dioxol-5-yl-5-methyl-3-(4-methyl-pyridin-2-ylamino)-dihydrofuran-2-on,
5-Methyl-3-(4-methyl-pyridin-2-ylamino)-5-(5,6,7,8-tetrahydro-naphthalin-2-yl)-dihydro-furan-2-on,
5-Benzo[1,3]dioxol-5-yl-5-methyl-3-(5-methyl-pyridin-2-ylamino)-dihydrofuran-2-on,
5-Benzo[1,3]dioxol-5-yl-5-methyl-3-(6-methyl-pyridin-2-ylamino)-dihydrofuran-2-on,
3-(5-Brom-3-nitro-pyridin-2-ylamino)-5-methyl-5-(5,6,7,8-tetrahydronaphthalin-2-yl)-dihydro-furan-2-on,
3-(5-Brom-3-nitro-pyridin-2-ylamino)-5-isopropyl-5-phenyl-dihydro-furan-2-on,
5-Isopropyl-3-(5-nitro-pyridin-2-ylamino)-5-phenyl-dihydro-furan-2-on,
5-Methyl-5-naphthalin-2-yl-3-(5-nitro-pyridin-2-ylamino)-dihydro-furan-2-on,
5-Isopropyl-5-phenyl-3-(pyrimidin-2-ylamino)-dihydro-furan-2-on,
3-[5-(4-Iocl-phenyl)-5-methyl-2-oxo-tetrahydro-furan-3-ylamino]-1H-pyrazol-4-carbonsäureethylester,
5-(4-Brom-phenyl)-3-(5-brom-pyridin-2-ylamino)-5-methyl-dihydro-furan-2-on,
3-(3-Brom-5-methyl-pyridin-2-ylamino)-5-methyl-5-naphthalin-1-yl-dihydrofuran-2-on,
5-Methyl-5-naphthalin-1-yl-3-(6-propyl-pyridin-2-ylamino)-dihydro-furan-2-on,
5-(3-Chlor-phenyl)-5-methyl-3-(6-propyl-pyridin-2-ylamino)-dihydro-furan-2-on,
5-(3-Chlor-phenyl)-5-methyl-3-(4-methyl-3-nitro-pyridin-2-ylamino)-dihydrofuran-2-on,
5-(4-Brom-phenyl)-5-methyl-3-(4-methyl-3-nitro-pyridin-2-ylamino)-dihydrofuran-2-on,
3-(5-Brom-6-methyl-pyridin-2-ylamino)-5-methyl-5-naphthalin-1-yl-dihydrofuran-2-on,
3-(5-Brom-6-methyl-pyridin-2-ylamino-5-(4-iodo-phenyl)-5-methyl-dihydrofuran-2-on,
3-(3-Benzyloxy-pyridin-2-ylamino)-5-(4-iodo-phenyl)-5-methyl-dihydro-furan-2-on,
3-(3-Benzyloxy-pyridin-2-ylamino)-5-(4-brom-phenyl)-5-methyl-dihydrofuran-2-on,
3-(4,6-Dimethyl-pyridin-2-ylamino)-5-(4-iodo-phenyl)-5-methyl-dihydrofuran-2-on,
5-(3-Chlor-phenyl)-3-(4,6-dimethyl-pyridin-2-ylamino)-5-methyl-dihydrofuran-2-on,
5-(4-Brom-phenyl)-5-methyl-3-(3-methyl-pyridin-2-ylamino)-dihydro-furan-2-on,
5-(4-Brom-phenyl)-5-methyl-3-(4-methyl-pyridin-2-ylamino)-dihydro-furan-2-on,
2-[5-(3,5-Dimethoxy-phenyl-5-methyl-2-oxo-tetrahydro-furan-3-ylamino]-4-propyl-pyrimidin-5-carbonsäureethylester,
3-(4-Brom-1H-pyrazol-3-ylamino)-5-(3,5-dimethoxy-phenyl)-5-methyldihydro-furan-2-on,
3-(4-Brom-1H-pyrazol-3-ylamino)-5-(2-methoxy-phenyl)-5-methyl-dihydrofuran-2-on,
3-[5-(2,5-Dimethoxy-phenyl)-5-methyl-2-oxo-tetrahydro-furan-3-ylamino]-1H-pyrazol-4-carbonitril,
3-[5-(2,5-Dimethoxy-phenyl)-5-methyl-2-oxo-tetrahydro-furan-3-ylamino]-5-methylsulfanyl-1H-pyrazol-4-carbonitril,
5-(2,5-Dimethoxy-phenyl)-5-methyl-3-(pyridin-2-ylamino)-dihydro-furan-2-on,
5-(2-Methoxy-phenyl)-5-methyl-3-(pyridin-2-ylamino)-dihydro-furan-2-on,
3-(3-Chlor-5-trifluormethyl-pyridin-2-ylamino)-5-(3,5-dimethoxy-phenyl)-5-methyl-dihydro-furan-2-on,
3-(3-Chlor-5-trifluormethyl-pyridin-2-ylamino)-5-(2,5-dimethoxy-phenyl)-5-methyl-dihydro-furan-2-on,
3-(3,5-Dichlor-pyridin-2-ylamino)-5-(2-methoxy-phenyl)-5-methyl-dihydrofuran-2-on,
3-(3-Chlor-5-trifluormethyl-pyridin-2-ylamino)-5-(2,4-dimethoxy-phenyl)-5-methyl-dihydro-furan-2-on,
3-(4,6-Dimethyl-pyridin-2-ylamino)-5-(3-methoxy-phenyl)-5-methyl-dihydrofuran-2-on,
3-(4,6-Dimethyl-pyridin-2-ylamino)-5-(4-methoxy-phenyl)-5-methyl-dihydrofuran-2-on,
5-(3,4-Dimethoxy-phenyl)-3-(4,6-dimethyl-pyridin-2-ylamino)-5-methyldihydro-furan-2-on,
5-(4-Methoxy-phenyl)-5-methyl-3-(4-methyl-pyridin-2-ylamino)-dihydrofuran-2-on,
5-(2,5-Dimethoxy-phenyl)-5-methyl-3-(pyrazin-2-ylamino)-dihydro-furan-2-on und
5-Methylsulfanyl-3-(2-oxo-5-phenyl-5-propyl-tetrahydro-furan-3-ylamino)-1 H-pyrazol-4-carbonitril
sowie deren entsprechende physiologisch verträgliche Salze, vorzugsweise deren Hydrochloride, eingesetzt wird.

6. Verwendung wenigstens einer substituierten γ-Lactonverbindung der allgemeinen Formel I gemäß einem der Ansprüche 1 bis 5 zur Herstellung eines Arzneimittels zur Behandlung von septischem Schock.

7. Verwendung wenigstens einer substituierten γ-Lactonverbindung der allgemeinen Formel I gemäß einem der Ansprüche 1 bis 5 zur Herstellung eines Arzneimittels zur Behandlung von neurodegenerativen Erkrankungen.

8. Verwendung gemäß Anspruch 7 zur Herstellung eines Arzneimittels zur Behandlung von multipler Sklerose.

9. Verwendung gemäß Anspruch 7 zur Herstellung eines Arzneimittels zur Behandlung von Morbus Parkinson.

10. Verwendung gemäß Anspruch 7 zur Herstellung eines Arzneimittels zur Behandlung von Morbus Alzheimer.

11. Verwendung gemäß Anspruch 7 zur Herstellung eines Arzneimittels zur Behandlung von Morbus Huntington.

12. Verwendung wenigstens einer substituierten γ-Lactonverbindung der allgemeinen Formel I gemäß einem der Ansprüche 1 bis 5 zur Herstellung eines Arzneimittels zur Behandlung von Entzündungen.

13. Verwendung wenigstens einer substituierten γ-Lactonverbindung der allgemeinen Formel I gemäß einem der Ansprüche 1 bis 5 zur Herstellung eines Arzneimittels zur Behandlung von Entzündungsschmerz.

14. Verwendung wenigstens einer substituierten γ-Lactonverbindung der allgemeinen Formel I gemäß einem der Ansprüche 1 bis 5 zur Herstellung eines Arzneimittels zur Behandlung von cerebraler Ischämie.

15. Verwendung wenigstens einer substituierten γ-Lactonverbindung der allgemeinen Formel I gemäß einem der Ansprüche 1 bis 5 zur Herstellung eines Arzneimittels zur Behandlung von Diabetes.

16. Verwendung wenigstens einer substituierten γ-Lactonverbindung der allgemeinen Formel I gemäß einem der Ansprüche 1 bis 5 zur Herstellung eines Arzneimittels zur Behandlung von Meningitis.

17. Verwendung wenigstens einer substituierten γ-Lactonverbindung der allgemeinen Formel I gemäß einem der Ansprüche 1 bis 5 zur Herstellung eines Arzneimittels zur Behandlung von Arteriosklerose.

18. Verwendung wenigstens einer substituierten γ-Lactonverbindung der allgemeinen Formel I gemäß einem der Ansprüche 1 bis 5 zur Herstellung eines Arzneimittels zur Wundheilung.

19. Verwendung wenigstens einer substituierten γ-Lactonverbindung der allgemeinen Formel I gemäß einem der Ansprüche 1 bis 5 zur Herstellung eines Arzneimittels zur Behandlung von Krebserkrankungen.

20. Verwendung wenigstens einer substituierten γ-Lactonverbindung der allgemeinen Formel I gemäß einem der Ansprüche 1 bis 5 zur Herstellung eines Amneimittels zur Behandlung von Pilzerkrankungen.

## Claims

1. Use of at least one substituted γ-lactone compound of the general formula I, 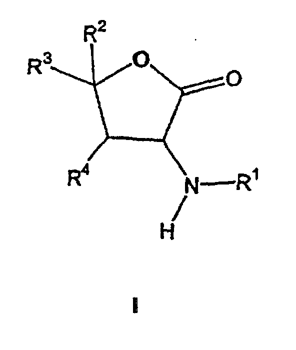 in which
R¹ denotes an optionally at least mono-substituted 2-pyridyl, 2-pyrimidyl, 2-pyrazolyl, 2-quinolinyl or 2-pyrazinyl residue, which may also be fused with a saturated or at least partially unsaturated hydrocarbon ring system,
R² denotes an optionally at least mono-substituted, saturated, branched or unbranched aliphatic C₁₋₁₀ residue or an optionally at least mono-substituted, at least partially unsaturated, branched or unbranched aliphatic C₂₋₁₀ residue,
R³ denotes an optionally at least mono-substituted aryl residue,
R⁴ denotes H,
or
R³ and R⁴ together denote an optionally at least mono-substituted, saturated or at least mono-unsaturated aliphatic C₃₋₇ residue, with the proviso that the residue R² in this case denotes an optionally at least mono-substituted aryl residue, an optionally at least mono-substituted, saturated, branched or unbranched aliphatic C₁₋₁₀ residue or an optionally at least mono-substituted, at least partially unsaturated, branched or unbranched aliphatic C₂₋₁₀ residue,
in the form of the racemates, diastereomers or enantiomers thereof as a free base or of a corresponding physiologically acceptable salt for the production of a pharmaceutical preparation for the treatment of migraine.

2. Use according to claim 1, **characterised in that** R¹ denotes an optionally at least mono-substituted 2-pyridyl residue, which may also be fused with a saturated or at least partially unsaturated hydrocarbon ring system, preferably denotes a 2-pyridyl residue which is substituted at least in position 4.

3. Use according to claim 1 or 2, **characterised in that** R² denotes an optionally at least mono-substituted, branched or unbranched C₁₋₆ residue.

4. Use according to one of claims 1 to 3, **characterised in that** R³ denotes an optionally at least mono-substituted aryl residue and R⁴ denotes H.

5. Use according to one of claims 1 to 4, **characterised in that** the compound used of the general formula I according to claim 1 comprises at least one compound selected from the group consisting of
5-(2,4-Dimethyl-phenyl)-3-(8-hydroxy-quinolin-2-ylamino)-5-methyl-dihydro-furan-2-one,
5-(3,4-Dimethyl-phenyl)-3-(4,6-dimethyl-pyridin-2-ylamino)-5-methyl-dihydro-furan-2-one,
5-(2,4-Dimethyl-phenyl)-3-(4,6-dimethyl-pyridin-2-ylamino)-5-methyl-dihydro-furan-2-one,
5-(4-Cyclohexyl-phenyl)-3-(4,6-dimethyl-pyridin-2-ylamino)-5-methyl-dihydro-furan-2-one,
5-(3,5-Dimethyl-phenyl)-3-(4,6-dimethyl-pyridin-2-ylamino)-5-methyl-dihydro-furan-2-one,
5-(3,4-Dimethyl-phenyl)-5-methyl-3-(4-methyl-pyridin-2-ylamino)-dihydro-furan-2-one,
5-(2,4-Dimethyl-phenyl)-5-methyl-3-(4-methyl-pyridin-2-ylamino)-dihydro-furan-2-one,
5-(4-Cyclohexyl-phenyl)-5-methyl-3-(4-methyl-pyridin-2-ylamino)-dihydro-furan-2-one,
5-Methyl-3-(quinolin-2-ylamino)-5-m-tolyl-dihydro-furan-2-one,
3-(4,6-Dimethyl-pyridin-2-ylamino)-5-methyl-5-p-tolyl-dihydro-furan-2-one,
3-(4,6-Dimethyl-pyridin-2-ylamino)-5-methyl-5-m-tolyl-dihydro-furan-2-one,
3-(4,6-Dimethyl-pyridin-2-ylamino)-5-(4-ethoxy-phenyl)-5-methyl-dihydro-furan-2-one,
4-[4-(3-Bromo-5-methyl-pyridin-2-ylamino)-2-methyl-5-oxo-tetrahydro-furan-2-yl]-benzonitrile,
3-(3-Bromo-5-methyl-pyridin-2-ylamino)-5-(4-tert-butyl-phenyl)-5-methyl-dihydro-furan-2-one,
5-(4-tert-Butyl-phenyl)-5-methyl-3-(6-propyl-pyridin-2-ylamino)-dihydro-furan-2-one,
5-(4-tert-Butyl-phenyl)-5-methyl-3-(4-methyl-3-nitro-pyridin-2-ylamino)-dihydro-furan-2-one,
3-(5-Bromo-6-methyl-pyridin-2-ylamino)-5-methyl-5-(4-phenoxy-phenyl)-dihydro-furan-2-one,
5-(4-tert-Butyl-phenyl)-5-methyl-3-(3-nitro-pyridin-2-ylamino)-dihydro-furan-2-one,
3-(3-Benzyloxy-pyridin-2-ylamino)-5-methyl-5-(4-phenoxy-phenyl)-dihydro-furan-2-one,
3-(3-Benzyloxy-pyridin-2-ylamino)-5-(4-tert-butyl-phenyl)-5-methyl-dihydro-furan-2-one,
3-(4,6-Dimethyl-pyridin-2-ylamino)-5-methyl-5-(4-phenoxy-phenyl)-dihydro-furan-2-one,
5-(4-tert-Butyl-phenyl)-3-(4,6-dimethyl-pyridin-2-yl-amino)-5-methyl-dihydrofuran-2-one,
5-Methyl-3-(4-methyl-pyridin-2-ylamino)-5-(4-phenoxy-phenyl)-dihydro-furan-2-one,
5-(4-tert-Butyl-phenyl)-5-methyl-3-(4-methyl-pyridin-2-ylamino)-dihydro-furan-2-one,
4-[4-(5-Bromo-3-nitro-pyridin-2-ylamino)-2-methyl-5-oxo-tetrahydro-furan-2-yl]-benzonitrile,
4-[4-(5-Bromo-pyrimidin-2-ylamino)-2-methyl-5-oxo-tetrahydro-furan-2-yl]-benzonitrile,
5-Benzo[b]thiophen-2-yl-5-methyl-3-(6-propyl-pyridin-2-ylamino)-dihydro-furan-2-one,
3-(4,6-Dimethyl-pyridin-2-ylamino)-5-(4-isopropyl-phenyl)-5-methyl-dihydro-furan-2-one,
5-Benzofuran-2-yl-3-(4,6-dimethyl-pyridin-2-ylamino)-5-methyl-dihydro-furan-2-one,
5-Benzo[b]thiophen-2-yl-5-methyl-3-(4-methyl-pyridin-2-ylamino)-dihydro-furan-2-one,
5-Benzofuran-2-yl-5-methyl-3-(4-methyl-pyridin-2-ylamino)-dihydro-furan-2-one,
3-(5-Benzo[1,3]dioxol-5-yl-5-methyl-2-oxo-tetrahydro-furan-3-ylamino)-1H-pyrazole-4-carbonitrile,
3-(5-Benzo[1,3]dioxol-5-yl-5-methyl-2-oxo-tetrahydro-furan-3-ylamino)-1H-pyrazole-4-carboxylic acid ethyl ester,
5-Benzo[1,3]dioxol-5-yl-5-methyl-3-(3-nitro-pyridin-2-ylamino)-dihydro-furan-2-one,
3-(4,6-Dimethyl-pyridin-2-ylamino)-5-methyl-5-(5,6,7,8-tetrahydro-naphthalen-2-yl)-dihydro-furan-2-one,
3-(4,6-Dimethyl-pyridin-2-ylamino)-5-methyl-5-naphthalen-2-yl-dihydro-furan-2-one,
5-Benzo[1,3]dioxol-5-yl-5-methyl-3-(4-methyl-pyridin-2-ylamino)-dihydro-furan-2-one,
5-Methyl-3-(4-methyl-pyridin-2-ylamino)-5-(5,6,7,8-tetrahydro-naphthalen-2-yl)-dihydro-furan-2-one,
5-Benzo[1,3]dioxol-5-yl-5-methyl-3-(5-methyl-pyridin-2-ylamino)-dihydro-furan-2-one,
5-Benzo[1,3]dioxol-5-yl-5-methyl-3-(6-methyl-pyridin-2-ylamino)-dihydro-furan-2-one,
3-(5-Bromo-3-nitro-pyridin-2-ylamino)-5-methyl-5-(5,6,7,8-tetrahydro-naphthalen-2-yl)-dihydro-furan-2-one,
3-(5-Bromo-3-nitro-pyridin-2-ylamino)-5-isopropyl-5-phenyl-dihydro-furan-2-one,
5-Isopropyl-3-(5-nitro-pyridin-2-ylamino)-5-phenyl-dihydro-furan-2-one,
5-Methyl-5-naphthalen-2-yl-3-(5-nitro-pyridin-2-ylamino)-dihydro-furan-2-one,
5-Isopropyl-5-phenyl-3-(pyrimidin-2-ylamino)-dihydro-furan-2-one,
3-[5-(4-Iodo-phenyl)-5-methyl-2-oxo-tetrahydro-furan-3-ylamino]-1H-pyrazole-4-carboxylic acid ethyl ester,
5-(4-Bromo-phenyl)-3-(5-bromo-pyridin-2-ylamino)-5-methyl-dihydro-furan-2-one,
3-(3-Bromo-5-methyl-pyridin-2-ylamino)-5-methyl-5-naphthalen-1-yl-dihydro-furan-2-one,
5-Methyl-5-naphthalen-1-yl-3-(6-propyl-pyridin-2-ylamino)-dihydro-furan-2-one,
5-(3-Chloro-phenyl)-5-methyl-3-(6-propyl-pyridin-2-ylamino)-dihydro-furan-2-one,
5-(3-Chloro-phenyl)-5-methyl-3-(4-methyl-3-nitro-pyridin-2-ylamino)-dihydro-furan-2-one,
5-(4-Bromo-phenyl)-5-methyl-3-(4-methyl-3-nitro-pyridin-2-ylamino)-dihydro-furan-2-one,
3-(5-Bromo-6-methyl-pyridin-2-ylamino)-5-methyl-5-naphthalen-1-yl-dihydro-furan-2-one,
3-(5-Bromo-6-methyl-pyridin-2-ylamino)-5-(4-iodo-phenyl)-5-methyl-dihydro-furan-2-one,
3-(3-Benzyloxy-pyridin-2-ylamino)-5-(4-iodo-phenyl)-5-methyl-dihydro-furan-2-one,
3-(3-Benzyloxy-pyridin-2-ylamino)-5-(4-bromo-phenyl)-5-methyl-dihydro-furan-2-one,
3-(4,6-Dimethyl-pyridin-2-ylamino)-5-(4-iodo-phenyl)-5-methyl-dihydro-furan-2-one,
5-(3-Chloro-phenyl)-3-(4,6-dimethyl-pyridin-2-ylamino)-5-methyl-dihydro-furan-2-one,
5-(4-Bromo-phenyl)-5-methyl-3-(3-methyl-pyridin-2-ylamino)-dihydro-furan-2-one,
5-(4-Bromo-phenyl)-5-methyl-3-(4-methyl-pyridin-2-ylamino)-dihydro-furan-2-one,
2-[5-(3,5-Dimethoxy-phenyl)-5-methyl-2-oxo-tetrahydro-furan-3-ylamino]-4-propyl-pyrimidine-5-carboxylic acid ethyl ester,
3-(4-Bromo-1H-pyrazol-3-ylamino)-5-(3,5-dimethoxy-phenyl)-5-methyl-dihydro-furan-2-one,
3-(4-Bromo-1H-pyrazol-3-ylamino)-5-(2-methoxy-phenyl)-5-methyl-dihydro-furan-2-one,
3-[5-(2,5-Dimethoxy-phenyl)-5-methyl-2-oxo-tetrahydro-furan-3-ylamino]-1H-pyrazole-4-carbonitrile,
3-[5-(2,5-Dimethoxy-phenyl)-5-methyl-2-oxo-tetrahydro-furan-3-ylamino]-5-methylsulfanyl-1H-pyrazole-4-carbonitrile,
5-(2,5-Dimethoxy-phenyl)-5-methyl-3-(pyridin-2-ylamino)-dihydro-furan-2-one,
5- (2-Methoxy-phenyl) -5-methyl-3- (pyridin-2-ylamino)-dihydro-furan-2-one,
3-(3-Chloro-5-trifluoromethyl-pyridin-2-ylamino)-5-(3,5-dimethoxy-phenyl)-5-methyl-dihydro-furan-2-one,
3-(3-Chloro-5-trifluoromethyl-pyridin-2-ylamino)-5-(2,5-dimethoxy-phenyl)-5-methyl-dihydro-furan-2-one,
3-(3,5-Dichloro-pyridin-2-ylamino)-5-(2-methoxy-phenyl)-5-methyl-dihydro-furan-2-one,
3-(3-Chloro-5-trifluoromethyl-pyridin-2-ylamino)-5-(2,4-dimethoxy-phenyl)-5-methyl-dihydro-furan-2-one,
3-(4,6-Dimethyl-pyridin-2-ylamino)-5-(3-methoxy-phenyl)-5-methyl-dihydro-furan-2-one,
3-(4,6-Dimethyl-pyridin-2-ylamino)-5-(4-methoxy-phenyl)-5-methyl-dihydro-furan-2-one,
5-(3,4-Dimethoxy-phenyl)-3-(4,6-dimethyl-pyridin-2-ylamino)-5-methyl-dihydro-furan-2-one,
5-(4-Methoxy-phenyl)-5-methyl-3-(4-methyl-pyridin-2-ylamino)-dihydro-furan-2-one,
5-(2,5-Dimethoxy-phenyl)-5-methyl-3-(pyrazin-2-ylamino)-dihydro-furan-2-one and 5-Methylsulfanyl-3-(2-oxo-5-phenyl-5-propyl-tetrahydro-furan-3-ylamino)-1H-pyrazole-4-carbonitrile
and the corresponding physiologically acceptable salts thereof, preferably the hydrochlorides thereof.

6. Use of at least one substituted γ-lactone compound of the general formula I according to one of claims 1 to 5 for the production of a pharmaceutical preparation for the treatment of septic shock.

7. Use of at least one substituted γ-lactone compound of the general formula I according to one of claims 1 to 5 for the production of a pharmaceutical preparation for the treatment of neurodegenerative diseases.

8. Use according to claim 7 for the production of a pharmaceutical preparation for the treatment of multiple sclerosis.

9. Use according to claim 7 for the production of a pharmaceutical preparation for the treatment of Parkinson's disease.

10. Use according to claim 7 for the production of a pharmaceutical preparation for the treatment of Alzheimer's disease.

11. Use according to claim 7 for the production of a pharmaceutical preparation for the treatment of Huntington's chorea.

12. Use of at least one substituted γ-lactone compound of the general formula I according to one of claims 1 to 5 for the production of a pharmaceutical preparation for the treatment of inflammation.

13. Use of at least one substituted γ-lactone compound of the general formula I according to one of claims 1 to 5 for the production of a pharmaceutical preparation for the treatment of inflammatory pain.

14. Use of at least one substituted γ-lactone compound of the general formula I according to one of claims 1 to 5 for the production of a pharmaceutical preparation for the treatment of cerebral ischaemia.

15. Use of at least one substituted γ-lactone compound of the general formula I according to one of claims 1 to 5 for the production of a pharmaceutical preparation for the treatment of diabetes.

16. Use of at least one substituted γ-lactone compound of the general formula I according to one of claims 1 to 5 for the production of a pharmaceutical preparation for the treatment of meningitis.

17. Use of at least one substituted γ-lactone compound of the general formula I according to one of claims 1 to 5 for the production of a pharmaceutical preparation for the treatment of arteriosclerosis.

18. Use of at least one substituted γ-lactone compound of the general formula I according to one of claims 1 to 5 for the production of a pharmaceutical preparation for wound healing.

19. Use of at least one substituted γ-lactone compound of the general formula I according to one of claims 1 to 5 for the production of a pharmaceutical preparation for the treatment of neoplastic diseases.

20. Use of at least one substituted γ-lactone compound of the general formula I according to one of claims 1 to 5 for the production of a pharmaceutical preparation for the treatment of fungal diseases.

## Revendications

1. Utilisation d'au moins un composé γ-lactone substitué de formule générale I 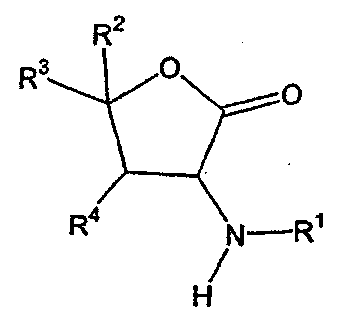 dans laquelle
R¹ représente un radical 2-pyridyle, 2-pyrimidyle, 2-pyrazolyle, 2-quinolinyle ou 2-pyrazinyle éventuellement au moins monosubstitué, qui peut également être condensé avec un système cyclique hydrocarboné saturé ou au moins partiellement insaturé,
R² représente un radical aliphatique en C₁₋₁₀ saturé, ramifié ou non ramifié, éventuellement au moins monosubstitué, ou un radical aliphatique en C₂₋₁₀ ramifié ou non ramifié, au moins partiellement insaturé, éventuellement au moins monosubstitué,
R³ représente un radical aryle éventuellement au moins monosubstitué,
R⁴ représente H,
ou
R³ et R⁴ représentent ensemble un radical aliphatique en C₃₋₇ saturé ou au moins mono-insaturé, éventuellement au moins monosubstitué, étant entendu que le radical R² représente en ce cas un radical aryle éventuellement au moins monosubstitué, un radical aliphatique en C₁₋₁₀ saturé, ramifié ou non ramifié, éventuellement au moins monosubstitué, ou un radical aliphatique en C₂₋₁₀ ramifié ou non ramifié, au moins partiellement insaturé, éventuellement au moins monosubstitué,
sous forme de leurs racémiques, diastéréoisomères ou énantiomères sous forme de base libre ou d'un sel physiologiquement acceptable correspondant, pour la fabrication d'un médicament destiné au traitement de la migraine.

2. Utilisation selon la revendication 1, **caractérisée en ce que** R¹ représente un radical 2-pyridyle éventuellement au moins monosubstitué, qui peut également être condensé avec un système cyclique hydrocarboné saturé ou au moins partiellement insaturé, de préférence un radical 2-pyridyle au moins substitué en position 4.

3. Utilisation selon la revendication 1 ou 2, **caractérisée en ce que** R² représente un radical en C₁₋₁₆ ramifié ou non ramifié, éventuellement au moins monosubstitué.

4. Utilisation selon l'une quelconque des revendications 1 à 3, **caractérisée en ce que** R³ représente un radical aryle éventuellement au moins monosubstitué et R⁴ représente H.

5. Utilisation selon l'une quelconque des revendications 1 à 4, **caractérisée en ce qu'**on utilise en tant que composé de formule générale I selon la revendication 1 au moins un composé choisi dans le groupe constitué par
la 5-(2,4-diméthylphényl)-3-(8-hydroxyquinolin-2-ylamino)-5-méthyldihydrofuran-2-one,
la 5-(3,4-diméthylphényl)-3-(4,6-diméthylpyridin-2-ylamino)-5-méthyldihydrofuran-2-one,
la 5-(2,4-diméthylphényl)-3-(4,6-diméthylpyridin-2-ylamino)-5-méthyldihydrofuran-2-one,
la 5-(4-cyclohexylphényl)-3-(4,6-diméthylpyridin-2-ylamino)-5-méthyldihydrofuran-2-one,
la 5-(3,5-diméthylphényl)-3-(4,6-diméthylpyridin-2-ylamino)-5-méthyldihydrofuran-2-one,
la 5-(3,4-diméthylphényl)-5-méthyl-3-(4-méthyl-pyridin-2-ylamino)- dihydrofuran-2-one,
la 5-(2,4-diméthylphényl)-5-méthyl-3-(4-méthyl-pyridin-2-ylamino)dihydrofuran-2-one,
la 5-(4-cyclohexylphényl)-5-méthyl-3-(4-méthylpyridin-2-ylamino)dihydrofuran-2-one,
la 5-méthyl-3-(quinolin-2-ylamino)-5-m-tolyl-dihydrofuran-2-one,
la 3-(4,6-diméthylpyridin-2-ylamino)-5-méthyl-5-p-tolyldihydrofuran-2-one,
la 3-(4,6-diméthylpyridin-2-ylamino)-5-méthyl-5-m-tolyldihydrofuran-2-one,
la 3-(4,6-diméthylpyridin-2-ylamino)-5-(4-éthoxy-phényl)-5-méthyldihydrofuran-2-one,
le 4-[4-(3-bromo-5-méthylpyridin-2-ylamino)-2-méthyl-5-oxotétrahydrofuran-2-yl]benzonitrile,
la 3-(3-bromo-5-méthylpyridin-2-ylamino)-5-(4-tert-butylphényl)-5-méthyldihydrofuran-2-one,
la 5-(4-tert-butylphényl)-5-méthyl-3-(6-propyl-pyridin-2-ylamino)dihydrofuran-2-one,
la 5-(4-tert-butylphényl)-5-méthyl-3-(4-méthyl-3-nitropyridin-2-ylamino)dihydrofuran-2-one,
la 3-(5-bromo-6-méthylpyridin-2-ylamino)-5-méthyl-5-(4-phénoxyphényl)dihydrofuran-2-one,
la 5-(4-tert-butylphényl)-5-méthyl-3-(3-nitro-pyridin-2-ylamino)dihydrofuran-2-one,
la 3-(3-benzyloxypyridin-2-ylamino)-5-méthyl-5-(4-phénoxyphényl)dihydrofuran-2-one,
la 3-(3-benzyloxypyridin-2-ylamino)-5-(4-tert-butylphényl)-5-méthyldihydrofuran-2-one,
la 3-(4,6-diméthylpyridin-2-ylamino)-5-méthyl-5-(4-phénoxyphényl)dihydrofuran-2-one,
la 5-(4-tert-butylphényl)-3-(4,6-diméthylpyridin-2-ylamino)-5-méthyldihydrofuran-2-one,
la 5-méthyl-3-(4-méthylpyridin-2-ylamino)-5-(4-phénoxyphényl)dihydrofuran-2-one,
la 5-(4-tert-butylphényl)-5-méthyl-3-(4-méthyl-pyridin-2-ylamino)dihydrofuran-2-one,
le 4-[4-(5-bromo-3-nitropyridin-2-ylamino)-2-méthyl-5-oxotétrahydrofuran-2-yl]benzonitrile,
le 4-[4-(5-bromopyrimidin-2-ylamino)-2-méthyl-5-oxotétrahydrofuran-2-yl]benzonitrile,
la 5-benzo[b]thiophén-2-yl-5-méthyl-3-(6-propyl-pyridin-2-ylamino)dihydrofuran-2-one,
la 3-(4,6-diméthylpyridin-2-ylamino)-5-(4-iso-propylphényl)-5-méthyldihydrofuran-2-one,
la 5-benzofuran-2-yl-3-(4,6-diméthylpyridin-2-yl-amino)-5-méthyldihydrofuran-2-one,
la 5-benzo[b]thiophén-2-yl-5-méthyl-3-(4-méthyl-pyridin-2-ylamino)dihydrofuran-2-one,
la 5-benzofuran-2-yl-5-méthyl-3-(4-méthylpyridin-2-ylamino)dihydrofuran-2-one,
le 3-(5-benzo[1,3]dioxol-5-yl-5-méthyl-2-oxotétrahydrofuran-3-ylamino)-1H-pyrazol-4-carbonitrile,
le 3-(5-benzo[1,3]dioxol-5-yl-5-méthyl-2-oxotétrahydrofuran-3-ylamino)-1H-pyrazol-4-carboxylate d'éthyle,
la 5-benzo[1,3]dioxol-5-yl-5-méthyl-3-(3-nitropyridin-2-ylamino)dihydrofuran-2-one,
la 3-(4,6-diméthylpyridin-2-ylamino)-5-méthyl-5-(5,6,7,8-tétrahydronaphtalén-2-yl)dihydrofuran-2-one,
la 3-(4,6-diméthylpyridin-2-ylamino)-5-méthyl-5-naphtalén-2-yldihydrofuran-2-one,
la 5-benzo[1,3]dioxol-5-yl-5-méthyl-3-(4-méthylpyridin-2-ylamino)dihydrofuran-2-one,
la 5-méthyl-3-(4-méthylpyridin-2-ylamino)-5-(5,6,7,8-tétrahydronaphtalén-2-yl)dihydrofuran-2-one,
la 5-benzo[1,3]dioxol-5-yl-5-méthyl-3-(5-méthylpyridin-2-ylamino)dihydrofuran-2-one,
la 5-benzo[1,3]dioxol-5-yl-5-méthyl-3-(6-méthylpyridin-2-ylamino)dihydrofuran-2-one,
la 3-(5-bromo-3-nitropyridin-2-ylamino)-5-méthyl-5-(5,6,7,8-tétrahydronaphtalén-2-yl)dihydrofuran-2-one,
la 3-(5-bromo-3-nitropyridin-2-ylamino)-5-iso-propyl-5-phényldihydrofuran-2-one,
la 5-isopropyl-3-(5-nitropyridin-2-ylamino)-5-phényldihydrofuran-2-one,
la 5-méthyl-5-naphtalén-2-yl-3-(5-nitropyridin-2-ylamino)dihydrofuran-2-one,
la 5-isopropyl-5-phényl-3-(pyrimidin-2-ylamino)-dihydrofuran-2-one,
le 3-[5-(4-iodophényl)-5-méthyl-2-oxotétrahydrofuran-3-ylamino]-1H-pyrazol-4-carboxylate d'éthyle,
la 5-(4-bromophényl)-3-(5-bromopyridin-2-ylamino)-5-méthyldihydrofuran-2-one,
la 3-(3-bromo-5-méthylpyridin-2-ylamino)-5-méthyl-5-naphtalén-1-yldihydrofuran-2-one,
la 5-méthyl-5-naphtalén-1-yl-3-(6-propylpyridin-2-ylamino)dihydrofuran-2-one,
la 5-(3-chlorophényl)-5-méthyl-3-(6-propylpyridin-2-ylamino)dihydrofuran-2-one,
la 5-(3-chlorophényl)-5-méthyl-3-(4-méthyl-3-nitropyridin-2-ylamino)dihydrofuran-2-one,
la 5-(4-bromophényl)-5-méthyl-3-(4-méthyl-3-nitropyridin-2-ylamino)dihydrofuran-2-one,
la 3-(5-bromo-6-méthylpyridin-2-ylamino)-5-méthyl-5-naphtalén-1-yldihydrofuran-2-one,
la 3-(5-bromo-6-méthylpyridin-2-ylamino)-5-(4-iodo-phényl)-5-méthyldihydrofuran-2-one,
la 3-(3-benzyloxypyridin-2-ylamino)-5-(4-iodo-phényl)-5-méthyldihydrofuran-2-one,
la 3-(3-benzyloxypyridin-2-ylamino)-5-(4-bromo-phényl)-5-méthyldihydrofuran-2-one,
la 3-(4,6-diméthylpyridin-2-ylamino)-5-(4-iodo-phényl)-5-méthyldihydrofuran-2-one,
la 5-(3-chlorophényl)-3-(4,6-diméthylpyridin-2-yl-amino)-5-méthyldihydrofuran-2-one,
la 5-(4-bromophényl)-5-méthyl-3-(3-méthylpyridin-2-ylamino)dihydrofuran-2-one,
la 5-(4-bromophényl)-5-méthyl-3-(4-méthylpyridin-2-ylamino)dihydrofuran-2-one,
le 2-[5-(3,5-diméthoxyphényl)-5-méthyl-2-oxotétrahydrofuran-3-ylamino]-4-propylpyrimidin-5-carboxylate d'éthyle
la 3-(4-bromo-1H-pyrazol-3-ylamino)-5-(3,5-diméthoxyphényl)-5-méthyldihydrofuran-2-one,
la 3-(4-bromo-1H-pyrazol-3-ylamino)-5-(2-méthoxyphényl)-5-méthyldihydrofuran-2-one,
le 3-[5-(2,5-diméthoxyphényl)-5-méthyl-2-oxotétrahydrofuran-3-ylamino]-1H-pyrazol-4-carbonitrile,
le 3-[5-(2,5-diméthoxyphényl)-5-méthyl-2-oxotétrahydrofuran-3-ylamino]-5-méthylsulfanyl-1H-pyrazol-4-carbonitrile,
la 5-(2,5-diméthoxyphényl)-5-méthyl-3-(pyridin-2-ylamino)dihydrofuran-2-one;
la 5-(2-méthoxyphényl)-5-méthyl-3-(pyridin-2-yl-amino)dihydrofuran-2-one,
la 3-(3-chloro-5-trifluorométhylpyridin-2-ylamino)-5-(3,5-diméthoxyphényl)-5-méthyldihydrofuran-2-one,
la 3-(3-chloro-5-trifluorométhylpyridin-2-ylamino)-5-(2,5-diméthoxyphényl)-5-méthyldihydrofuran-2-one,
la 3-(3,5-dichloropyridin-2-ylamino)-5-(2-méthoxyphényl)-5-méthyldihydrofuran-2-one,
la 3-(3-chloro-5-trifluorométhylpyridin-2-ylamino)-5-(2,4-diméthoxyphényl)-5-méthyldihydrofuran-2-one,
la 3-(4,6-diméthylpyridin-2-ylamino)-5-(3-méthoxyphényl)-5-méthyldihydrofuran-2-one,
la 3-(4,6-diméthylpyridin-2-ylamino)-5-(4-méthoxyphényl)-5-méthyldihydrofuran-2-one,
la 5-(3,4-diméthoxyphényl)-3-(4,6-diméthylpyridin-2-ylamino)-5-méthyldihydrofuran-2-one,
la 5-(4-méthoxyphényl)-5-méthyl-3-(4-méthylpyridin-2-ylamino)dihydrofuran-2-one,
la 5-(2,5-diméthoxyphényl)-5-méthyl-3-(pyrazin-2-ylamino)dihydrofuran-2-one et
le 5-méthylsulfanyl-3-(2-oxo-5-phényl-5-propyltétrahydrofuran-3-ylamino)-1H-pyrazol-4-carbonitrile
ainsi que leurs sels physiologiquement acceptables correspondants, de préférence leurs chlorhydrates.

6. Utilisation d'au moins un composé γ-lactone substitué de formule générale I selon l'une quelconque des revendications 1 à 5, pour la fabrication d'un médicament destiné au traitement du choc septique.

7. Utilisation d'au moins un composé γ-lactone substitué de formule générale I selon l'une quelconque des revendications 1 à 5, pour la fabrication d'un médicament destiné au traitement de maladies neuro-dégénératives.

8. Utilisation selon la revendication 7, pour la fabrication d'un médicament destiné au traitement de la sclérose en plaques.

9. Utilisation selon la revendication 7, pour la fabrication d'un médicament destiné au traitement de la maladie de Parkinson.

10. Utilisation selon la revendication 7, pour la fabrication d'un médicament destiné au traitement de la maladie d'Alzheimer.

11. Utilisation selon la revendication 7, pour la fabrication d'un médicament destiné au traitement de la chorée de Huntington.

12. Utilisation d'au moins un composé γ-lactone substitué de formule générale I selon l'une quelconque des revendications 1 à 5, pour la fabrication d'un médicament destiné au traitement d'inflammations.

13. Utilisation d'au moins un composé γ-lactone substitué de formule générale I selon l'une quelconque des revendications 1 à 5, pour la fabrication d'un médicament destiné au traitement de la douleur due à une inflammation.

14. Utilisation d'au moins un composé γ-lactone substitué de formule générale I selon l'une quelconque des revendications 1 à 5, pour la fabrication d'un médicament destiné au traitement de l'ischémie cérébrale.

15. Utilisation d'au moins un composé γ-lactone substitué de formule générale I selon l'une quelconque des revendications 1 à 5, pour la fabrication d'un médicament destiné au traitement du diabète.

16. Utilisation d'au moins un composé γ-lactone substitué de formule générale I selon l'une quelconque des revendications 1 à 5, pour la fabrication d'un médicament destiné au traitement de la méningite.

17. Utilisation d'au moins un composé γ-lactone substitué de formule générale I selon l'une quelconque des revendications 1 à 5, pour la fabrication d'un médicament destiné au traitement de l'artério-sclérose.

18. Utilisation d'au moins un composé γ-lactone substitué de formule générale I selon l'une quelconque des revendications 1 à 5, pour la fabrication d'un médicament destiné à la cicatrisation.

19. Utilisation d'au moins un composé γ-lactone substitué de formule générale I selon l'une quelconque des revendications 1 à 5, pour la fabrication d'un médicament destiné au traitement de maladies cancéreuses.

20. Utilisation d'au moins un composé γ-lactone substitué de formule générale I selon l'une quelconque des revendications 1 à 5, pour la fabrication d'un médicament destiné au traitement de mycoses.
